(19) Europäisches Patentamt
European Patent Office
Office européen des brevets

(11) EP 2 275 564 A1

(12) EUROPEAN PATENT APPLICATION

(43) Date of publication:
**19.01.2011 Bulletin 2011/03**

(51) Int Cl.:
***C12N 15/82*** *(2006.01)* *C12N 15/53* *(2006.01)*

(21) Application number: **09165807.0**

(22) Date of filing: **17.07.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **Freie Universität Berlin**
**14195 Berlin (DE)**

(72) Inventor: **The designation of the inventor has not yet been filed**

(74) Representative: **Gulde Hengelhaupt Ziebig & Schneider**
**Patentanwälte - Rechtsanwälte**
**Wallstrasse 58/59**
**10179 Berlin (DE)**

# (54) Means and method for the production of transgenic plants that are resistant to clubroot

(57) The present invention refers to an isolated nucleic acid, comprising a) at least one promoter sequence which is predominantly active in the hypocotyls compared to shoot apex tissue of a plant; and b) a nucleic acid sequence encoding for a protein that is able to decrease a cytokinin status in a plant tissue; wherein the nucleic acid sequence encoding said protein is located in 3'-position to the at least one promoter and wherein the at least one promoter sequence and the nucleic acid sequence encoding said protein are functionally linked with one another.

EP 2 275 564 A1

## Description

The clubroot disease of plants of the family *Brassicaceae* is caused by the obligate biotrophic protist *Plasmodiophora brassicae.* In the past few years clubroot has become a very important disease in rapeseed and other *Brassica* vegetable production, especially in Canada, Korea, Japan and China.

The interaction of the obligate biotrophic pathogen with its host, including *Arabidopsis thaliana,* leads to the formation of root galls (also called clubs) which creates a strong metabolic sink and influences growth and development of the host plants. The susceptible *A. thaliana* ecotype Col-0 develops large clubs in the main root and hypocotyls. Clubs can be extended to petioles and the basis of the stalk. Plant growth is retarded and seed set is reduced. Ultimately, the plant succumbs to the loss of water and nutrients from the root and from redirection of assimilates from leaves to roots (Siemens et al, 2002, Journal of Phytopathology, 150, 592-605).

It has been shown previously that plant hormones are involved in pathogenesis. In particular, the cytokinin status is relevant and reduction of overall cytokinin content by constitutive expression of a cytokinin-degrading cytokinin oxidase/dehydrogenase enzyme under a systemic promoter causes resistance to clubroot disease (Siemens et al., 2006, Molecular Plant-Microbe Interactions, 19, 480-494). Transgenic plants overexpressing cytokinin oxidase/dehydrogenase constitutively were disease resistant (tolerant) to all tested pathotypes of *Plasmodiophora brassicae.* This finding indicates the importance of cytokinin as a key factor in clubroot disease development. Histological analysis of the transgenic plants revealed an inhibition of the development of the pathogen. Four weeks after infection, the roots of cytokinin oxidase/dehydrogenase overexpressing lines were colonised by vegetative secondary plasmodia of the pathogen, but hyperplasia and hypertrophy was limited and only rarely spores were detected. Whereas in control plants, mature spores were already found 17 days after infection. These data demonstrated that the cytokinin status of the host plant has a major role in determining the success of the pathogen infection.

However a major drawback of constitutive overexpression of cytokinin oxidase/dehydrogenase has been identified (Siemens et al. 2006). Due to the systemic overexpression of the cytokinin degrading enzymes, the overall systemic cytokinin status of the transgenic plants is reduced. This systemic reduction of the cytokinin status causes a strong reduction of shoot growth. Inhibition of shoot growth is a disadvantageous phenotypic trait, which massively impacts economical and commercial usefulness of these transgenic plants.

It is an object of the invention to diminish or to avoid one or more of the drawbacks of the state of the art. In particular it is an object of the present invention to provide means and methods to produce transgenic plants which are resistant to clubroot disease and show sufficient shoot growth.

This object is achieved by the present invention as set out in detail below.

In a first aspect of the present invention, an isolated nucleic acid is provided, comprising:

a) at least one promoter sequence which is predominantly active in the hypocotyls compared to shoot apex tissue of a plant, preferably compared to any other aerial tissue of a plant; and

b) a nucleic acid sequence encoding for a protein that is able to decrease a cytokinin status, preferably a cytokinin level, in a plant tissue;

wherein the nucleic acid sequence encoding said protein is located in 3'-position (i.e. downstream) to the at least one promoter and wherein the at least one promoter sequence and the nucleic acid sequence encoding said protein are functionally linked with one another.

In a second aspect the invention provides a transgenic expression cassette for the expression of nucleic acids, wherein the transgenic expression cassette of the invention comprises an isolated nucleic acid according to the present invention. The transgenic expression cassette of the invention is designed such that it mediates the transgenic expression of the nucleic acid sequence encoding for a protein that is able to decrease a cytokinin status, preferably a cytokinin level, in a plant tissue under the control of the at least one promoter in a host organism, preferably a plant cell.

In a third aspect of the invention, a vector is provided comprising an isolated nucleic acid according to the invention or a transgenic expression cassette of the invention.

It was surprisingly found that transgenic expression of a protein that is able to decrease a cytokinin status, preferably a cytokinin level, in a plant tissue predominantly in the hypocotyls compared to shoot apex tissue of said transgenic plant, leads to transgenic plants exhibiting resistance to clubroot disease, while shoot growth is preserved to an extent allowing economic cultivation of transgenic plants. These findings demonstrate for the first time that not control of the overall cytokinin status of a plant is decisive whether the plant is resistant to clubroot disease, but that it is sufficient to reduce the cytokinin status, preferably the cytokinin level, predominantly in the hypocotyls or in the hypocotyls and the root, while cytokinin status in the shoot apex is affected to a lesser extent.

The isolated nucleic acid of the invention comprises at least one promoter sequence which is predominantly active in the hypocotyls compared to shoot apex tissue of a plant. The hypocotyls are a part of the plant that is located between the roots and the cotyledons. The term "shoot apex tissue" denotes any shoot apex tissue of a plant that is formed above ground after the hypocotyls and the cotyledons.

According to the present invention any promoter sequence can be used to produce an isolated nucleic acid of the invention, provided that the promoter sequence is predominantly active in the hypocotyls compared to shoot apex tissue of a plant. The term "active in the hypocotyls" is to be understood such that the promoter facilitates the expression of a gene that is functionally linked to said promoter at least in a tissue of plant hypocotyls. The expression "predominantly active" is to be understood that the expression level of a gene under control of such a promoter is higher in the tissue in which the promoter is predominantly active than in another tissue to which promoter activity is compared to. According to the present invention, a promoter sequence is predominantly active in the hypocotyls compared to shoot apex tissue of a plant, if the expression level conferred by said promoter sequence is higher in the hypocotyls than in shoot apex tissue, even more preferably compared to any other aerial tissue of a plant. Preferably the expression level conferred by said promoter is at least twice as high in the hypocotyls than in shoot apex tissue. More preferably the expression level conferred by said promoter is at least twice as high in the hypocotyls than in any other aerial tissue of a plant. The at least one promoter of the isolated nucleic acid of the invention can additionally be predominantly active in root tissue compared to shoot apex tissue of a plant. The skilled person is aware of a number of methods to determine expression level conferred by a promoter. Since for the purpose of the invention, it is only important that the at least one promoter confers an expression level in the hypocotyls that is higher than in shoot apex tissue, it does not matter which method is used as long as the same method is used to determine promoter activity in both tissues, the hypocotyls and shoot apex tissue. E.g. the expression level can be determined as absolute expression level per gram of tissue or per number of cells. The expression level can also be determined as relative expression level compared to a commonly expressed gene or protein.

The skilled person is well aware of such promoter sequences and has no difficulties in determining whether a given nucleic acid sequence exhibits above mentioned properties. A very convenient tool for identifying suitable promoter sequences is the Arabidopsis eFP Browser accessible under "http://bar.utoronto.ca/efp/cgibin/efpWeb.cgi". With this tool, the skilled person can easily identify promoter sequences that are predominantly active in the hypocotyls as defined above.

In particular the at least one promoter sequence can be selected from the nonlimiting examples of promoter sequences listed below in Table 1 or from a reverse complementary sequence thereof.

*Table 1: Selected promoter sequences suitable for use in an isolated nucleic acid of the invention.*

| Promoter of the gene | Accession number | SEQ ID No. | Relative expression level [arbitrary units] according to eFP | | | |
|---|---|---|---|---|---|---|
| | | | hypocotyls | Shoot apex, inflorescence | Shoot apex, transition | Shoot apex, vegetative |
| PYK10 | At3G09260 | SEQ ID No. 1 | 2983 | 13 | 50 | 1135 |
| AP2 domain-cont. trans. factor | At5G61590 | SEQ ID No. 2 | 504 | 90 | 74 | 138 |
| HSF A7A | At3G51910 | SEQ ID No. 3 | 1710 | 3 | 34 | 8 |
| Trans. factor TINY | At5G25810 | SEQ ID No. 4 | 107 | 20 | 2 | 3 |
| MYB122 | At1 G74080 | SEQ ID No. 5 | 6 | 6 | 3 | 6 |
| | At3G16390 | SEQ ID No. 12 | 2493 | 245 | 560 | 795 |
| | At3G16460 | SEQ ID No. 13 | 1602 | 10 | 21 | 229 |
| | At4G37410 | SEQ ID No. 14 | 2181 | 16 | 177 | 306 |
| | At3G16450 | SEQ ID No. 15 | 1951 | 12 | 14 | 163 |
| | At5G64100 | SEQ ID No. 16 | 117 | 2 | 8 | 10 |
| | At3G16430 | SEQ ID No. 17 | 2852 | 14 | 168 | 1263 |

(continued)

| Promoter of the gene | Accession number | SEQ ID No. | Relative expression level [arbitrary units] according to eFP | | | |
|---|---|---|---|---|---|---|
| | | | hypocotyls | Shoot apex, inflorescence | Shoot apex, transition | Shoot apex, vegetative |
| | At3G16420 | SEQ ID No. 18 | 2852 | 14 | 168 | 1263 |
| | At1 G03230 | SEQ ID No. 19 | 909 | 350 | 503 | 433 |
| | At1 G03220 | SEQ ID No. 20 | 909 | 350 | 503 | 433 |
| | At1 G73260 | SEQ ID No. 21 | 90 | 2 | 4 | 4 |
| | At3G15950 | SEQ ID No. 22 | 1140 | 63 | 163 | 368 |
| | At4G12550 | SEQ ID No. 23 | 68 | 1 | 1 | 1 |
| | At4G27652 | SEQ ID No. 24 | 271 | 17 | 6 | 74 |

The at least one promoter sequence of an isolated nucleic acid of the present invention can be selected from:

i) SEQ ID Nos. 1, 2, 3, 4, 5, 12, 13, 14, 15, 16, 17, 18, 19, 20, 21, 22, 23 or 24 or a reverse complement thereof, preferably SEQ ID Nos. 1, 2, 3, 4, 12, 13, 14, 15, 16, 17, 18, 19, 20, 22 or 24 or a reverse complement thereof ; or
ii) from functionally equivalent sequences which have at least 60% homology with one of the sequence of i) over a sequence segment of at least 100 base pairs and which function as promoter sequence which is predominantly active in the hypocotyls compared to shoot apex tissue of a plant; or
iii) from functionally equivalent fragments of one of the sequences of i) or ii) with a length of at least 100 base pairs and which function as promoter sequence which is predominantly active in the hypocotyls compared to shoot apex tissue of a plant.

The isolated nucleic acid of the invention can comprise functionally equivalent sequences or fragments of one of the promoters with the SEQ ID Nos. 1, 2, 3, 4, 5, 12, 13, 14, 15, 16, 17, 18, 19, 20, 21, 22, 23 or 24 or a reverse complement thereof, preferably SEQ ID Nos. 1, 2, 3, 4, 12, 13, 14, 15, 16, 17, 18, 19, 20, 22 or 24 or a reverse complement thereof.

To prepare such functionally equivalent sequences or fragments, it is possible, for example, to delete nonessential sequences of a promoter according to the invention without adversely affecting the abovementioned essential properties to a significant extent. Such deletion variants constitute functionally equivalent sequences or fragments of the promoter described by SEQ ID Nos. 1, 2, 3, 4, 5, 12, 13, 14, 15, 16, 17, 18, 19, 20, 21, 22, 23 or 24 or a reverse complement thereof. The delimitation of the promoter sequence to certain, essential regulatory regions can be carried out for example with the aid of search routines for the search of promoter elements. Frequently, certain promoter elements are amassed in the regions which are relevant for the promoter activity. This analysis can be carried out for example with computer programs such as the program PLACE ("Plant Cis-acting Regulatory DNA Elements") (Higo K et al. (1999) Nucl Acids Res 27(1): 297-300) or the BIOBASE database "Transfac" (Biologische Datenbanken GmbH, Braunschweig). Preferably, the functionally equivalent sequences or fragments of one of the promoters according to the invention - for example one of the promoters described by SEQ ID Nos. 1, 2, 3, 4, 5, 12, 13, 14, 15, 16, 17, 18, 19, 20, 21, 22, 23 or 24 or a reverse complement thereof - comprise at least 100 base pairs, very especially preferably at least 300 base pairs, most preferably at least 500 base pairs being identical with a part of the respective promoter sequence described by one of SEQ ID Nos. 1, 2, 3, 4, 5, 12, 13, 14, 15, 16, 17, 18, 19, 20, 21, 22, 23 or 24 or a reverse complement thereof or being at least 60% homologous with a part of the respective promoter sequence described by one of SEQ ID Nos. 1, 2, 3, 4, 5, 12, 13, 14, 15, 16, 17, 18, 19, 20, 21, 22, 23 or 24 or a reverse complement thereof. In a preferred embodiment, the functionally equivalent sequence or fragment comprises the 3' region of one of the promoters described by SEQ ID Nos. 1, 2, 3, 4, 5, 12, 13, 14, 15, 16, 17, 18, 19, 20, 21, 22, 23 or 24 or a reverse complement thereof, the fragment length which is in each case preferred being calculated in 5' direction upstream from the transcription start or translation start ("ATG" codon).

The functionally equivalent sequences or fragments function as promoter sequence which is predominantly active in the hypocotyls compared to shoot apex tissue of a plant. Thus, the promoter activity of functionally equivalent sequences or fragments is preferably essentially the same as the promoter activity of the respective promoter described by one of SEQ ID Nos. 1, 2, 3, 4, 5, 12, 13, 14, 15, 16, 17, 18, 19, 20, 21, 22, 23 or 24 or a reverse complement thereof. A promoter activity is referred to as essentially the same when the transcription of a certain gene to be expressed under the control of, for example, a functional equivalent fragment of one of the promoter sequences described by SEQ ID Nos. 1, 2, 3, 4, 5, 12, 13, 14, 15, 16, 17, 18, 19, 20, 21, 22, 23 or 24 or a reverse complement thereof, under otherwise unchanged conditions, is higher in hypocotyls than in shoot apical tissue, preferably twice as high in hypocotyls than in shoot apex tissue of a plant.

The expression level of a functionally equivalent sequence or fragment can deviate both downward and/or upward compared with the respective promoter of one of SEQ ID Nos. 1, 2, 3, 4, 5, 12, 13, 14, 15, 16, 17, 18, 19, 20, 21, 22, 23 or 24 or a reverse complement thereof. Preferred in this context are those sequences or fragments whose expression level, measured on the basis of the transcribed mRNA or the subsequently translated protein, under conditions which are otherwise unchanged, differs quantitatively by not more than 50%, preferably 25%, particularly preferably 10%, from a comparison value obtained with the respective promoter described by one of SEQ ID Nos. 1, 2, 3, 4, 5, 12, 13, 14, 15, 16, 17, 18, 19, 20, 21, 22, 23 or 24 or a reverse complement thereof. Especially preferred sequences or fragments are those whose expression level, measured on the basis of the transcribed mRNA or the subsequently translated protein, under conditions which are otherwise unchanged, exceeds quantitatively a comparison value obtained with the respective promoter described by one of SEQ ID Nos. 1, 2, 3, 4, 5, 12, 13, 14, 15, 16, 17, 18, 19, 20, 21, 22, 23 or 24 or a reverse complement thereof by more than 50%, preferably 100%, especially preferably 500%, very especially preferably 1000%. Preferred as comparison value is the expression level of the mRNAs of PYK10 expressed naturally by the promoter with SEQ ID No. 1, or the protein resulting there from. Furthermore preferred as comparison value is the expression level obtained with any defined nucleic acid sequence under the control of the promoter with SEQ ID No. 1, preferably those nucleic acid sequences which encode readily quantifiable proteins. Very especially preferred in this context are reporter proteins (Schenborn E & Groskreutz D (1999) Mol Biotechnol 13(1):29-44) such as the "green fluorescence protein" (GFP) (Chui W L et al. (1996) Gurr Biol 6:325-330; Leffel S M et al. (1997) Biotechniques. 23(5): 912-8), the chloramphenicol transferase, a luciferase (Millar et al. (1992) Plant Mol Biol Rep 10:324-414) or the β-glucuronidase; β-glucuronidase is very especially preferred (Jefferson et al. (1987) EMBO J. 6:3901-3907).

Functional equivalent sequences or fragments also comprise natural or artificial mutations of the promoter sequence described by one of SEQ ID Nos. 1, 2, 3, 4, 5, 12, 13, 14, 15, 16, 17, 18, 19, 20, 21, 22, 23 or 24 or a reverse complement thereof. Mutations comprise substitutions, additions, deletions, inversions or insertions of one or more nucleotide residues. Thus, for example, the present invention also comprises those nucleotide sequences which are obtained by modification of the PYK10 promoter of SEQ ID NO: 1. The aim of such a modification may be the further delimitation of the sequence present therein or else, for example, the introduction or removal of restriction endonuclease cleavage sites, the removal of superfluous DNA or the addition of further sequences, for example further regulatory sequences. Where insertions, deletions or substitutions such as, for example, transitions and transversions, are suitable it is possible to use techniques known per se, such as in-vitro mutagenesis, primer repair, restriction or ligation. Transition means a base pair exchange of a purine/pyrimidine pair into another purine/pyrimidine pair (for example A-T for G-C). Transversion means a base pair exchange of a purine/pyrimidine pair for a pyrimidine/purine pair (e.g. A-T for T-A). Deletion means removal of one or more base pairs. Insertion means the introduction of one or more base pairs.

Complementary ends of the sequences or fragments can be made available for ligation by means of manipulations such as restriction, chewing back or filling in overhangs for what are known as blunt ends. Analogous results can also be obtained using the polymerase chain reaction (PCR) using specific oligonucleotide primers.

The isolated nucleic acid of the present invention can comprise functional equivalent sequences or fragments of one of the promoter sequences described by one of SEQ ID Nos. 1, 2, 3, 4, 5, 12, 13, 14, 15, 16, 17, 18, 19, 20, 21, 22, 23 or 24 or a reverse complement thereof, preferably one of SEQ ID Nos. 1, 2, 3, 4, 12, 13, 14, 15, 16, 17, 18, 19, 20, 22 or 24 or a reverse complement thereof. Said functional equivalent sequences or fragments have at least 60%, preferably at least 80%, especially preferably at least 90% homology with one of the sequence described by SEQ ID Nos. 1, 2, 3, 4, 5, 12, 13, 14, 15, 16, 17, 18, 19, 20, 21, 22, 23, 24 or a reverse complement thereof over a sequence segment of at least 100 base pairs, preferably at least 300 base pairs, very especially preferably at least 500 base pairs, most preferably over the entire sequence length, and have essentially the same promoter activity as the respective promoter sequence selected from SEQ ID Nos. 1, 2, 3, 4, 5, 12, 13, 14, 15, 16, 17, 18, 19, 20, 21, 22, 23 or 24 or a reverse complement thereof.

Homology between two nucleic acids is understood as meaning the identity of the nucleic acid sequence over the complete sequence length in each case, which is calculated by comparison with the aid of the GAP program algorithm (Wisconsin Package Version 10.0, University of Wisconsin, Genetics Computer Group (GCG), Madison, USA), setting the following parameters:

| | |
|---|---|
| Gap Weight: | 12 |
| Length Weight: | 4 |
| Average Match: | 2.912 |
| Average Mismatch: | -2.003 |

For example, a sequence which has at least 60% homology with the sequence of SEQ ID No. 1 on nucleic acid basis is understood as meaning a sequence which, upon comparison with the sequence SEQ ID No. 1 by the above program algorithm with the above set of parameters, has at least 60% homology.

Functional equivalent sequences or fragments also includes nucleic acid, preferably DNA, sequences which hybridize under standard conditions with the nucleic acid sequence encoding one of the promoters described in SEQ ID Nos. 1, 2, 3, 4, 5, 12, 13, 14, 15, 16, 17, 18, 19, 20, 21, 22, 23 or 24 or a reverse complement thereof or with the nucleic acid sequences complementary thereto, and which have substantially the same promoter properties. The term standard hybridization conditions is to be understood broadly and means both stringent and/or less stringent hybridization conditions. Such hybridization conditions are described inter alia in Sambrook J, Fritsch E F, Maniatis T et al., in Molecular Cloning-A Laboratory Manual, 2nd edition, Cold Spring Harbor Laboratory Press, 1989, pages 9.31-9.57 or in Current Protocols in Molecular Biology, John Wiley & Sons, N.Y. (1989), 6.3.1-6.3.6.

For example, the conditions during the washing step(s) can be selected from the range of conditions limited by those of low stringency (with approximately 2*SSC at 50 °C) and of high stringency (with approximately 0.2*SSC at 50 °C, preferably at 65 °C) (20*SSC: 0.3 M sodium citrate, 3 M NaCl, pH 7.0). In addition, the temperature during the washing step can be raised from low-stringency conditions at room temperature, approximately 22 °C., to more stringent conditions at approximately 65 °C. Both parameters, the salt concentration and the temperature, can be varied simultaneously, and it is also possible for one of the two parameters to be kept constant and only the other to be varied. It is also possible to employ denaturing agents such as, for example, form amide or SDS during the hybridization. Hybridization in the presence of 50% formamide is preferably carried out at 42 °C. Some exemplary conditions for hybridization and washing steps are given below:

(1) Hybridization conditions with for example

a) 4*SSC at 65 °C., or
b) 6*SSC, 0.5% SDS, 100 μg/ml denatured fragmented salmon sperm DNA at 65 °C., or
c) 4*SSC, 50% formamide, at 42 °C., or
d) 2* or 4*SSC at 50 °C. (low-stringency condition), or
e) 2* or 4*SSC, 30 to 40% formamide at 42 °C. (low-stringency condition), or
f) 6*SSC at 45 °C., or,
g) 0.05 M sodium phosphate buffer pH 7.0, 2 mM EDTA, 1 % BSA and 7% SDS.

(2) Washing steps with for example

a) 0.1 *SSC at 65 °C., or
b) 0.1*SSC, 0.5% SDS at 68 °C., or
c) 0.1*SSC, 0.5% SDS, 50% formamide at 42 °C., or
d) 0.2*SSC, 0.1 % SDS at 42 °C., or
e) 2*SSC at 65 °C. (low-stringency condition), or
f) 40 mM sodium phosphate buffer pH 7.0, 1 % SDS, 2 mM EDTA.

Methods for preparing functional equivalent sequences or fragments of the invention preferably comprise the introduction of mutations into one of the promoters described by SEQ ID Nos. 1, 2, 3, 4, 5, 12, 13, 14, 15, 16, 17, 18, 19, 20, 21, 22, 23 or 24 or a reverse complement thereof. Mutagenesis may be random, in which case the mutagenized sequences are subsequently screened for their properties by a trial and error procedure. Particularly advantageous selection criteria comprise for example the level of the resulting expression of the introduced nucleic acid sequence in the hypocotyls compared to shoot apex tissue. Methods for mutagenesis of nucleic acid sequences are known to the skilled worker and include by way of example the use of oligonucleotides with one or more mutations compared with the region to be mutated (e.g. in a site-specific mutagenesis). Primers with approximately 15 to approximately 75 nucleotides or more are typically employed, with preferably about 10 to about 25 or more nucleotide residues being located on both sides of the sequence to be modified. Details and procedure for said mutagenesis methods are familiar to the skilled worker (Kunkel et al. (1987) Methods Enzymol 154:367-382; Tomic et al. (1990) Nucl Acids Res 12:1656; Upender et al. (1995) Biotechniques 18(1):29-30; U.S. Pat. No. 4,237,224). A mutagenesis can also be achieved by treating for example transgenic expression vectors comprising one of the nucleic acid sequences of the invention with mutagenizing agents such as hydroxylamine.

The isolated nucleic acid of the invention comprises a nucleic acid sequence encoding for a protein that is able to decrease a cytokinin status in a plant tissue. Preferably the isolated nucleic acid of the invention comprises a nucleic acid sequence encoding for a protein that is able to decrease a cytokinin level in a plant tissue. The term "cytokinin status" refers not only to the concentration of one or all of the cytokinin hormones as such (particularly referred to as "cytokinin level"), but further comprises the perception of a cytokinin by a plant cell and transmission of a cytokinin induced signal within a plant cell or between different plant cells of the same plant. A low cytokinin status thus means a reduced reaction of a cell or tissue to the hormone, which can be preferentially achieved by a lower hormone level, but also by lowering the hormone perception and/or its signal transduction.

Cytokinins (CK) are a class of plant hormones that can promote cell division. Cytokinins are primarily involved in cell division, differentiation, and other physiological processes. There are two types of cytokinins: adenine-type cytokinins represented by kinetin, zeatin and 6-benzylaminopurine, as well as phenylurea-type cytokinins like diphenylurea or thidiazuron (TDZ).

Proteins that are able to decrease a cytokinin status and/or particularly a cytokinin level in a plant tissue are well known to the skilled person. Such proteins encompass e.g. proteins that directly degrade one or more classes of cytokinins and as such directly decrease a cytokinin level. Alternatively such proteins may be involved in regulation of transcription or translation of proteins that directly degrade cytokinins and as such indirectly decrease a cytokinin level. Such proteins also encompass proteins which can inhibit or block the synthesis or the transport of cytokinins, e.g. by possessing a cytokinin synthetase inhibitory activity. A well known class of proteins that is able to decrease a cytokinin level in a plant tissue is the class of proteins with cytokinin oxidase/dehydrogenase activity. Alternatively, proteins that are able to decrease a cytokinin status in a plant tissue also comprise proteins that inhibit or block a cytokinin signalling pathway. Examples of such proteins are described in PCT/EP2007/008331, in particular the dominant negative regulator ARR1-SRDX is explicitly comprised.

The isolated nucleic acid sequence preferably comprises a nucleic acid sequence encoding for a protein with cytokinin oxidase/dehydrogenase activity, preferably a nucleic acid encoding a protein of the CKX family. The nucleic acid sequence encoding for a protein with cytokinin oxidase/dehydrogenase activity preferably is selected from nucleic acid sequences encoding CKX1, CKX2, CKX3, CKX4, CKX5, CKX6 or CKX7. Particularly preferred are nucleic acid sequences encoding for CKX family members derived from *Arabidopsis thaliana,* e.g. AtCKX1 denoted as SEQ ID No: 6, AtCKX2 denoted as SEQ ID No. 7, AtCKX3 denoted as SEQ ID No. 8, AtCKX4 denoted as SEQ ID No. 9, AtCKX5 denoted as SEQ ID No. 10, AtCKX6 denoted as SEQ ID No. 11 or AtCKX7 denoted as SEQ ID No. 29, particularly preferred is AtCKX1 denoted as SEQ ID No. 6 and/or AtCKX3 denoted as SEQ ID No. 8.

The isolated nucleic acid of the invention preferably comprises a nucleic acid sequence encoding for a protein that is able to decrease a cytokinin status, preferably a cytokinin level, in a plant tissue selected from:

i) one of SEQ ID Nos. 6, 7, 8, 9, 10, 11 or 29; or

ii) from sequences which have at least 60% homology, preferably at least 80% homology, more preferably at least 90% homology with one of the sequence of SEQ ID Nos. 6, 7, 8, 9, 10, 11 or 29 over a coding sequence segment of at least 300 base pairs, preferably over a coding sequence segment of at least 500 base pairs, more preferably over the whole coding sequence length, and which encode for a protein with cytokinin oxidase/dehydrogenase activity; or

iii) from sequences which hybridise under standard conditions with one of the nucleic acid sequences described in SEQ ID Nos. 6, 7, 8, 9, 10, 11 or 29 or with a nucleic acid sequences complementary thereto, and which encode for a protein with cytokinin oxidase/dehydrogenase activity.

Sequence homology is determined as explained in detail above.

The term standard hybridization conditions is to be understood broadly and means stringent and/or less stringent hybridization conditions. Such hybridization conditions are described exemplarily above.

The skilled person is well aware of means and methods to determine whether a given protein has cytokinin oxidase/dehydrogenase activity. A cytokinin dehydrogenase is an enzyme that catalyzes the chemical reaction:

$$\text{N6-dimethylallyladenine} + \text{acceptor} + H_2O \rightleftharpoons \text{adenine} + \text{3-methylbut-2-enal} + \text{reduced acceptor}$$

The 3 substrates of this enzyme are N6-dimethylallyladenine, acceptor, and $H_2O$, whereas its 3 products are adenine, 3-methylbut-2-enal, and reduced acceptor.

There is ample guidance in the literature how a given protein can be tested for such an activity, see e.g. EC 1.5.99.12. Preferably the term "cytokinin oxidase/dehydrogenase activity" encompasses the activity of a given protein to catalyse an oxidoreductase reaction with at least one of the cytokinins as substrate.

More preferably the term "cytokinin oxidase/dehydrogenase activity" encompasses the activity of a given protein to catalyse an oxidoreductase reaction with at least one of the cytokinins as substrate and with an activity of not less than 30% of the activity of AtCKX3, preferably of not less than 50% of the activity of AtCKX3.

In the isolated nucleic acid of the invention, the nucleic acid encoding a protein that is able to decrease a cytokinin status, preferably a cytokinin level, in a plant tissue is located downstream of the at least one promoter sequence in 3'-position to the at least one promoter.

The at least one promoter and the nucleic acid sequence encoding for a protein that is able to decrease a cytokinin status, preferably a cytokinin level, in a plant are functionally linked with one another. A functional linkage means, for example, the sequential arrangement of one of the at least one promoters according to the invention, of the nucleic acid sequence encoding the protein to be expressed transgenically and, if appropriate, of further regulatory elements such as e.g. a terminator, in such a way that each of the regulatory elements is able to fulfil its expected function in the transgenic expression of the nucleic acid sequence encoding the desired protein. This does not necessarily require a direct linkage in the chemical sense. Genetic control sequences such as, for example, enhancer sequences, can also exert their function on the target sequence from positions which are further removed, or indeed from other DNA molecules. In the isolated nucleic acid of the invention the nucleic acid sequence to be expressed transgenically is positioned downstream of the sequence which acts as the at least one promoter sequence so that both sequences are coupled covalently with one another. Preferably, the distance between the at least one promoter sequence and the nucleic acid sequence to be expressed transgenically is less than 200 base pairs, especially preferably less than 100 base pairs, very especially preferably less than 50 base pairs.

"Expression" means in this context the transcription of the nucleic acid sequence to be expressed transgenically, but can also include the translation of the transcribed RNA of the nucleic acid sequence to be expressed transgenically into a corresponding polypeptide.

"Transgenic" means-for example regarding a transgenic expression cassette, a transgenic expression vector, a transgenic organism or method for the transgenic expression of nucleic acids-all those constructs which are the result of transgenic methods, or all methods using them, in which an isolated nucleic acid of the invention is not located in their natural genetic environment or has been modified by transgenic methods, where the modification can be for example a substitution, addition, deletion, inversion or insertion of one or more nucleotide residues. Preferably, the at least one promoter sequence of the isolated nucleic acid according to the invention is heterologous with regard to the further nucleic acid sequence which is linked functionally with it and which is to be expressed transgenically. In this context, "heterologous" means that the further nucleic acid sequence does not comprise the coding sequence which is naturally under the control of said promoter.

"Natural genetic environment" means the natural chromosomal locus in the organism of origin or the presence in a genomic library. In the case of a genomic library, the natural genetic environment of the nucleic acid sequence is preferably retained at least in part. The environment flanks the nucleic acid sequence at least at one side and has a sequence length of at least 50 bp, preferably at least 500 bp, especially preferably at least 1000 bp, very especially preferably at least 5000 bp. A naturally occurring expression construct-for example the naturally occurring combination of the promoter of SEQ ID NO: 1 and the coding sequence of the PYK10 gene becomes a transgenic expression construct when this combination is modified by non-natural, synthetic ("artificial") methods such as, for example, an in-vitro mutagenesis. Such methods have been described (U.S. Pat. No. 5,565,350; WO 00/15815; see also hereinabove).

"Transgenic" with regard to an expression ("transgenic expression") preferably means all those expressions which have been carried out using a transgenic expression cassette, transgenic expression vector or transgenic organism, as defined hereinabove or below.

A functional linkage between the at least one promoter and the nucleic acid sequence to be expressed can be produced by means of conventional recombination and cloning techniques as are described, for example, in Maniatis T et al. (1989) Molecular Cloning: A Laboratory Manual, Cold Spring Harbor Laboratory, Cold Spring Harbor, N.Y. and in Silhavy T J et al. (1984) Experiments with Gene Fusions, Cold Spring Harbor Laboratory, Cold Spring Harbor, N.Y. and in Ausubel F M et al. (1987) Current Protocols in Molecular Biology, Greene Publishing Assoc. and Wiley Interscience. A method which is suitable for this purpose is, for example, the GATEWAY(TM) cloning technology (Invitrogen Inc.), which is based on recombination.

An isolated nucleic acid of the invention is prepared for example by fusing one of the promoters according to the invention with a nucleotide sequence encoding for a protein that is able to decrease a cytokinin status, preferably a cytokinin level, in a plant tissue and optionally with a terminator and/or polyadenylation signal. The combination of the PYK10 promoter with the sequence encoding a CKX protein is especially preferred in this context.

The isolated nucleic acid according to the invention can comprise further genetic control sequences, besides the at least one promoter sequence according to the invention.

The concept of the genetic control sequences is to be understood broadly and means all those sequences which have an effect on the origin or the function of the isolated nucleic acid or the transgenic expression cassette according to the invention. Genetic control sequences modify, for example, the transcription and/or translation in prokaryotic or eukaryotic organisms. Preferably, the isolated nucleic acid or the transgenic expression cassettes according to the invention comprise one of the promoters according to the invention 5'-upstream from the particular nucleic acid sequence to be expressed transgenically and a terminator sequence 3'-downstream as additional genetic control sequence, and, if appropriate, further customary regulatory elements, in each case functionally linked with the nucleic acid sequence to be expressed transgenically.

Genetic control sequences can also comprise further promoters, promoter elements or minimal promoters which are capable of modifying expression-controlling properties. It is thus possible, by means of genetic control sequences, that for example tissue-specific expression takes place in addition in dependence on certain stress factors. Such additional genetic control elements can be preferably DNA binding motifs for a suppressive transcription factor that is down-regulated endogenously in a plant in response to contact of a plant tissue with a biotrophic pathogen, in particular to *Plasmodiophora brassicae.* Suitable elements are described, for example, for water stress, abscisic acid (Lam E and Chua N H (1991) J Biol Chem 266(26):17131-17135) and heat stress (Schöffl F et al. (1989) Mol Gen Genet (217(2-3):246-53).

It is furthermore possible that further promoters which make possible expression in further plant tissues or in other organisms such as, for example, *E. coli* bacteria, are linked functionally with the nucleic acid sequence to be expressed. Suitable plant promoters are, in principle, all of the above-described promoters. For example, it is conceivable that a certain nucleic acid sequence is transcribed, in a plant tissue, as sense RNA, and translated into the corresponding protein, by one promoter (for example one of the promoters according to the invention), while the same nucleic acid sequence is, in a different tissue, transcribed into antisense RNA, and the corresponding protein is downregulated, by a different promoter with a different specificity. This can be carried out by means of a transgenic expression cassette according to the invention by positioning the one promoter upstream of the nucleic acid sequence to be expressed transgenically and the other promoter downstream of it.

Genetic control sequences furthermore also comprise the 5'-untranslated region, introns, the noncoding 3' region or else sequences of genes, preferably of genes encoding a protein that is able to decrease a cytokinin status, preferably a cytokinin level, in a plant tissue, more preferably encoding for a protein with cytokinin oxidase/dehydrogenase activity. It has been shown that 5'-untranslated sequences are capable of enhancing the transient expression of heterologous genes. Furthermore, they may promote tissue specificity (Rouster J et al. (1998) Plant J 15:435-440). Conversely, the 5'-untranslated region of the opaque-2 gene suppresses expression. Deletion of the region in question results in an increase in gene activity (Lohmer S et al. (1993) Plant Cell 5:65-73).

The isolated nucleic acid can advantageously comprise one or more of what are known as enhancer sequences in functional linkage with the promoter, which make increased transgenic expression of the nucleic acid sequence possible. Additional advantageous sequences can also be inserted at the 3' end of the nucleic acid sequences to be expressed transgenically, such as further regulatory elements or terminators. The nucleic acid sequences to be expressed transgenically can be present as one or more copies in one of the transgenic expression cassettes according to the invention.

Control sequences are furthermore understood as meaning those which make possible homologous recombination or insertion into the genome of a host organism, or which permit deletion from the genome. In the case of homologous recombination, one of the promoters according to the invention may be substituted for the natural promoter of a particular gene, for example. One of the promoters according to the invention can-as described above-be placed, by means of homologous recombination, before an endogenous target gene to be expressed transgenically, by linking the promoter with DNA sequences which are for example homologous to endogenous sequences located upstream of the reading frame of the target gene. Such sequences are to be understood as genetic control sequences. Methods such as the cre/lox technology permit tissue-specific, and in some circumstances inducible, deletion of the transgenic expression cassette from the genome of the host organism (Sauer B (1998) Methods (Duluth) 14(4):381-92). Here, certain flanking sequences are added to the target gene (lox sequences), which later make possible deletion by means of cre recombinase.

To select cells which have successfully undergone homologous recombination, or else transformation, it is, as a rule, necessary additionally to introduce a selectable marker (see hereinbelow). Homologous recombination is a relatively rare event in higher eukaryotes, in particular in plants. Random integrations into the host genome predominate. One possibility of deleting the randomly integrated sequences, and thus to increase the concentration of cell clones with a correct homologous recombination, is the use of a sequence-specific recombination system as described in U.S. Pat. No. 6,110,736.

Polyadenylation signals which are suitable as control sequences are plant polyadenylation signals and-preferably-those which essentially correspond to T-DNA polyadenylation signals from *Agrobacterium tumefaciens.* In a particularly preferred embodiment, the isolated nucleic acid or the transgenic expression cassette comprises a terminator sequence which is functional in plants. Terminator sequences which are functional in plants generally means those sequences which are capable of bringing about, in plants, the termination of the transcription of a DNA sequence. Examples of suitable terminator sequences are the OCS (octopine synthase) terminator and the NOS (nopalin synthase) terminator. However, plant terminator sequences are especially preferred. Plant terminator sequences generally refers to those sequences which are part of a natural plant gene. Especially preferred in this context is the terminator of the potato cathepsin D inhibitor gene or the terminator of the field bean storage protein gene VfLE1B3. These terminators are at least equivalent to the viral or T-DNA terminators described in the prior art.

The isolated nucleic acid or the transgenic expression cassettes according to the invention and vectors derived from them may comprise further functional elements. The term functional element is to be understood broadly and means all those elements which have an effect on the generation, multiplication or function of the transgenic expression cassettes according to the invention or on transgenic expression vectors or organisms derived from them. The following may be mentioned by way of example, but not by limitation:

1. Selection Markers

The term "selection marker" comprises not only positive selection markers, which confer a resistance to an antibiotic, herbicide or other biocide, but also negative selection markers, which confer a sensitivity to precisely the abovementioned, and also markers which confer a growth advantage to the transformed organism (for example by expression of key genes of cytokinin biosynthesis; Ebinuma H et al. (2000) Proc Natl Acad Sci USA 94:2117-2121). In the case of positive selection, only those organisms which express the selection marker in question thrive, while precisely these organisms die in the case of negative selection. The use of a positive selection marker is preferred in the generation of transgenic plants. Furthermore preferred is the use of selection markers which confer growth advantages. Negative selection markers can be used advantageously when the task at hand consists in eliminating certain genes or genome segments from an organism (for example for the purposes of a hybridization process).

i) Positive Selection Markers: The selectable marker introduced with the transgenic expression cassette confers resistance to a biocide, for example a herbicide (such as phosphinothricin, glyphosate or bromoxynil), a metabolic inhibitor (such as 2-deoxyglucose-6-phosphate; WO 98/45456) or an antibiotic (such as, for example, tetracyclins, ampicillin, kanamycin, G 418, neomycin, bleomycin or hygromycin) to the successfully transformed cells. The selection marker permits the selection of the transformed cells from untransformed cells (McCormick et al. (1986) Plant Cell Reports 5:81-84). Especially preferred selection markers are those which confer resistance to herbicides. Selection markers which may be mentioned by way of example are: genes which encode phosphinothricin acetyltransferases (PAT) (also referred to as Bialophos(R) resistance gene (bar)), which acetylate the free amino group of the glutamine synthase inhibitor phosphinothricin (PPT) and thus detoxify the PPT (de Block et al. (1987) EMBO J. 6:2513-2518; Vikkers JE et al. (1996) Plant Mol Biol Reporter 14:363-368; Thompson CJ et al. (1987) EMBO J. 6:2519-2523). The bar/PAT gene can be isolated for example from *Streptomyces hygroscopicus* or *S. viridochromogenes.* Such genes are known to the skilled worker (see e.g. U.S. Pat. No. 5,489,520). Synthetic genes are further described for expression in plastids. 5-Enolpyruvylshikimate-3-phosphate synthase genes (EPSP synthase genes), which confer resistance to Glyphosat(R) (N-(phosphonomethyl)glycin). The nonselective herbicide glyphosate has 5-enolpyruvyl-3-phosphoshikimate synthase (EPSPS) as molecular target. This enzyme has a key function in the biosynthesis of aromatic amino acids in plants (Steinrucken H C et al. (1980) Biochem Biophys Res Commun 94:1207-1212; Levin J G and Sprinson D B (1964) J Biol Chem 239:1142-1150; Cole D J (1985) Mode of action of glyphosate; A literature analysis, pp. 48-74. In: Grossbard E and Atkinson D (eds.). The herbicide glyphosate. Buttersworths, Boston). Glyphosate-tolerant EPSPS variants are preferably used as selection markers (Padgette S R et al. (1996). New weed control opportunities: development of soybeans with a Roundup Ready gene. In: Herbicide Resistant Crops (Duke S O, ed.), pp. 53-84. CRC Press, Boca Raton, Fla.; Saroha M K and Malik V S (1998) J Plant Biochemistry and Biotechnology 7:65-72). The EPSPS gene of *Agrobacterium sp.* strain CP4 has a natural tolerance to glyphosate which can be transferred to corresponding transgenic plants. The CP4 EPSPS gene has been cloned from *Agrobacterium sp.* strain CP4 (Padgette S R et al. (1995) Crop Science 35(5):145-1461). 5-Enolpyruvylshikimate-3-phosphate synthases which are glyphosate-tolerant, such as, for example, those described in U.S. Pat. No. 5,510,471; U.S. Pat. No. 5,776,760; U.S. Pat. No. 5,864,425; U.S. Pat. No. 5,633,435; U.S. Pat. No. 5,627,061; U.S. Pat. No. 5,463,175; EP 0 218 571, are preferred. The aroA gene is furthermore preferred; the gox gene (glyphosate oxidoreductase), which encodes the Glyphosat(R)-degrading enzymes. GOX (for example the glyphosate oxidoreductase from *Achromobacter sp.)* catalyzes the cleavage of a C-N bond in glyphosate, which is thus converted into aminomethylphosphonic acid (AMPA) and glyoxylate. GOX can thereby confer resistance to glyphosate (Padgette S R et al. (1996) J Nutr 126(3):702-16; Shah D et al. (1986) Science 233:478-481). The deh gene (encoding a dehalogenase which inactivates Dalapon(R); WO 99/27116); bxn genes, which encode Bromoxynil(R)-degrading nitrilase enzymes, for example the *Klebsiella ozanenae* nitrilase. Neomycin phosphotransferases (npt) confer resistance to antibiotics (aminoglycosides) such as neomycin, G418, hygromycin, paromomycin or kanamycin, by reducing their inhibitory action by means of a phosphorylation reaction. Especially preferred is the NPTII gene. Moreover, the gene is already a component in a large number of expression vectors and can be isolated from them using methods with which the skilled worker is familiar (such as, for example, polymerase chain reaction). The NPTII gene encodes an aminoglycoside 3'-O-phosphotransferase from E. coli, Tn5 (Beck et al. (1982) Gene 19 327-336). The DOG<R> I gene was isolated from the yeast *Saccharomyces cerevisiae* (EP 0 807 836) and it encodes a 2-deoxyglucose-6-phosphate phosphatase, which confers resistance to 2-DOG (Randez-Gil et al. (1995) Yeast 11:1233-1240; Sanz et al. (1994) Yeast 10:1195-1202). Sulfonylurea- and imidazolinone-inactivating acetolactate synthases, which confer resistance to imidazolinone/sulfonylurea herbicides. Examples which may be mentioned of imidazolinone herbicides are the active substances imazamethabenz-methyl, imazzamox, imazapyr, imazaquin and imazethapyr. Examples of sulfonylurea herbicides which may be mentioned are amidosulforon, azimsulfuron, chlorimuronethyl, chlorsulfuron, cinosulfuron, imazosulforon, oxasulforon, prosulforon, rimsulforon, sulfosulforon. The skilled worker is familiar with a large number of further active substances from the abovementioned classes. The Csr 1.2 gene of *Arabidopsis thaliana* (EC 4.1.3.18) is suitable for example (Sathasivan K et al. (1990) Nucleic Acids Res. 18(8):2188). Acetolactate synthases, which confer resistance to imidazolinone herbicides, are furthermore described. Hygromycin phosphotransferases which confer resistance to the antibiotic hygromycin. The gene is a component of a large number of expression vectors and can be isolated from them using methods with which the skilled worker is familiar (such as, for example, polymerase chain reaction). Genes for resistance to a) chloramphenicol (chloramphenicol acetyltransferase), b) tetracyclin; various resistance genes have been described. Moreover, the gene is already a component of a large number of expression vectors and can be isolated therefrom using methods known to the skilled worker (such as, for example, polymerase chain reaction) c) Streptomycin; various resistance genes have been described; d) Zeocin; the corresponding resistance gene is a component of a large number of cloning vectors and can be isolated from these using methods known to the skilled worker (such as, for example, polymerase chain reaction). e) Ampicillin ([beta]-lactamase gene; Datta N, Richmond M H. (1966) Biochem J 98(1):204-9; Heffron F et al. (1975) J. Bacteriol 122:250-256; the Amp gene was first cloned for generating the *E. coli* vector pBR322; Bolivar F et al. (1977) Gene 2:95-114). The gene is a component of a large number of cloning vectors and can be isolated from them using methods known to the skilled worker (such as, for example, polymerase chain reaction). Genes such as the isopentenyl transferase from *Agrobacterium tumefaciens* (strain: PO22). The ipt gene is a key enzyme of cytokinin biosynthesis. Its overexpression facilitates the regeneration of plants (for example selection on cytokinin-free medium). The method for utilizing the ipt gene has been described (Ebinuma H et al. (2000) Proc Natl Acad Sci USA 94:2117-2121; Ebinuma H et al. (2000) Selection of marker-free transgenic plants using the oncogenes (ipt, rol A, B, C) of Agrobacterium as selectable markers, In: Molecular Biology of Woody Plants. Kluwer Academic Publishers). Various other positive selection markers which confer a growth advantage to the transformed plants over nontransformed plants, and methods for their use, have been described, inter.alia, in EP-A 0 601 092. Examples which may be mentioned are β-glucuronidase (in conjunction with, for example, cytokinin glucuronide), mannose-6-phosphate isomerase (in conjunction with mannose), UDP-galactose 4-epimerase (in conjunction with, for example, galactose), with mannose-6-phosphate isomerase in conjunction with mannose being especially preferred.

ii) Negative Selection Markers: Negative selection markers make possible for example the selection of organisms with successfully deleted sequences which comprise the marker gene (Koprek T et al. (1999) The Plant Journal 19 (6):719-726). When carrying out a negative selection, for example a compound which otherwise has no disadvantageous effect on the plant is converted into a compound which is disadvantageous, for example owing to the negative selection marker introduced into the plant. Genes which have a disadvantageous effect per se are furthermore suitable. Negative selection markers which may be mentioned by way of example, but not by limitation, are thymidine kinase (TK), diphtheria toxin A fragment (DT-A), the coda gene product encoding a cytosine deaminase (Gleave A P et al. (1999) Plant Mol Biol 40(2):223-35; Perera R J et al. (1993) Plant Mol Biol 23(4): 793-799; Stougaard J (1993) Plant J 3:755-761), the cytochrome P450 gene (Koprek et al. (1999) Plant J 16:719-726), genes encoding a haloalkane dehalogenase (Naested H (1999) Plant J 18:571-576), the iaaH gene (Sundaresan V et al. (1995) Genes & Development 9:1797-1810) or the tms2 gene (Fedoroff N V & Smith D L (1993) Plant J 3:273-289).

2) Reporter Genes

Reporter genes encode readily quantifiable proteins which, via their colour or enzyme activity, allow an assessment of the transformation efficiency, the site or time of expression (see also Schenbron E, Groskreutz D. Mol Biotechnol. 1999; 13(1):29-44). Examples which may be mentioned are: "green fluorescence protein" (GFP) (Chui W L et al. (1996), Curr Biol 6:325-330; Leffel S M et al. (1997) Biotechniques 23(5):912-8; Sheen et al. (1995) Plant J 8(5): 777-784; Haseloff et al. (1997) Proc Natl Acad Sci USA 94(6):2122-2127; Reichel et al. (1996) Proc Natl Acad Sci USA 93(12):5888-5893; Tian et al. (1997) Plant Cell Rep 16:267-271; WO 97/41228). Chloramphenicol transferase (Fromm et al. (1985) Proc Natl Acad Sci USA 82:5824-5828), Luciferase (Millar et al. (1992) Plant Mol Biol Rep 10:324-414; Ow et al. (1986) Science 234:856-859); allows detection via bioluminescence. $\beta$-Galactosidase, encodes an enzyme for which a variety of chromogenic substrates are available. $\beta$-Glucuronidase (GUS) (Jefferson et al. (1987) EMBO J. 6: 3901-3907) or the uidA gene, which encodes an enzyme for a variety of chromogenic substrates. R-Locus gene product: protein which regulates the production of anthocyanine pigments (red coloration) in plant tissue and thus makes possible the direct analysis of the promoter activity without addition of further auxiliary substances or chromogenic substrates (Dellaporta et al. (1988) In: Chromosome Structure and Function: Impact of New Concepts, 18th Stadler Genetics Symposium, 11:263-282). Tyrosinase (Katz et al. (1983) J Gen Microbiol 129:2703-2714), an enzyme which oxidizes tyrosine to DOPA and dopaquinone, which subsequently form melanin, which can be detected readily. Aequorin (Prasher et al. (1985) Biochem Biophys Res Commun 126(3):1259-1268), can be used in the calcium-sensitive bioluminescence detection.

3) Replication Origins

Replication origins ensure the multiplication of the transgenic expression cassettes or transgenic expression vectors according to the invention in, for example, *E. coli* or agrobacteria. Examples which may be mentioned are OR1 (origin of DNA replication), the pBR322 ori or the P15A ori (Sambrook et al.: Molecular Cloning. A Laboratory Manual, 2<nd > ed. Cold Spring Harbor Laboratory Press, Cold Spring Harbor, N.Y., 1989). Examples of replication origins which are functional in Agrobacterium are pRK2, pRi, PVS1 or pSA.

4) Border Sequences

"Border sequences" (such as, for example, the right or left border of the T-DNA) make possible an agrobacteria-mediated transfer into plant cells for the transfer and integration into the plant genome.

5) Multiple Cloning Regions (MCS) permit and facilitate the insertion of one or more nucleic acid sequences.

The invention also relates to vectors which comprise the above-described isolated nucleic acid of the invention or the transgenic expression cassette of the invention. Vectors generally means structures which are capable of replication and which are preferably host-specific, and which make possible the uptake of nucleic acid sequences and their transfer into other cells. Examples of vectors can be plasmids, cosmids, phages, viruses or else agrobacteria. Vectors which are particularly suitable for the purposes of plant biotechnology are described herein below.

Another subject of the invention relates to transgenic organisms, transformed with at least one isolated nucleic acid of the invention or at least one transgenic expression cassette according to the invention or one transgenic expression vector according to the invention, and to cells, cell cultures, tissues, parts-such as, for example in the case of plant organisms, leaves, roots and the like-or propagation material derived from such organisms.

Organism, starting organisms or host organisms are understood as meaning prokaryotic or eukaryotic organisms such as, for example, microorganisms or plant organisms. Preferred microorganisms are bacteria, yeasts, algae or fungi.

Preferred bacteria are bacteria of the genus *Escherichia, Erwinia, Agrobacterium, Flavobacterium, Alcaligenes* or *cyanobacteria,* for example of the genus *Synechocystis.*

Especially preferred are microorganisms which are capable of infecting plants and thus of transferring the cassettes according to the invention. Preferred microorganisms are those from the genus *Agrobacterium* and in particular the species *Agrobacterium tumefaciens.*

Host or starting organisms which are preferred as transgenic organisms are, above all, plant organisms. Plant organisms generally means all those organisms which are capable of photosynthesis. Included as plant organisms within the scope of the invention are all genera and species of the higher and lower plants of the plant kingdom. The mature plants, seeds, tubers, beets/swollen tap roots, fruits, shoots and seedlings and also parts, propagation material and cultures, for example cell cultures, derived therefrom are also included. Mature plants means plants at any developmental stage beyond the seedling. Seedling means a young immature plant in an early developmental stage. Annual, perennial, monocotyledonous and dicotyledonous plants are preferred host organisms for preparing transgenic plants. Preference is given to plants of the following plant family: *Brassicaceae* in particular to plants of the genera *Brassica* and *Arabidopsis.*

The preparation of a transformed organism or of a transformed cell requires introducing the appropriate DNA into the appropriate host cell. A multiplicity of methods is available for this process which is referred to as transformation (see also Keown et al. 1990 Methods in Enzymology 185:527-537). Thus, by way of example, the DNA may be introduced directly by microinjection or by bombardment with DNA-coated microparticles. The cell may also be permeabilized chemically, for example using polyethylene glycol, so that the DNA can enter the cell via diffusion. The DNA may also be performed via protoplast fusion with other DNA-containing units such as minicells, cells, lysosomes or liposomes. Another suitable method for introducing DNA is electroporation in which the cells are reversibly permeabilized by an electric impulse.

In the case of plants, the methods described for transforming and regenerating plants from plant tissues or plant cells are utilized for transient or stable transformation. Suitable methods are especially protoplast transformation by polyethylene glycol-induced DNA uptake, the biolistic method using the gene gun, the "particle bombardment" method, electroporation, the incubation of dry embryos in DNA-containing solution and microinjection.

Apart from these "direct" transformation techniques, a transformation may also be carried out by bacterial infection by means of *Agrobacterium tumefaciens* or *Agrobacterium rhizogenes*. These strains contain a plasmid (Ti or Ri plasmid), a part of which (what is known as T-DNA) is transferred to the plant after infection with *Agrobacterium* and integrated into the genome of the plant cell. The *Agrobacterium*-mediated transformation is best suited to dicotyledonous plant cells, whereas the direct transformation techniques are suitable for any cell type.

A transgenic expression cassette of the invention may be introduced advantageously into cells, preferably into plant cells, by using vectors.

In an advantageous embodiment, the transgenic expression cassette is introduced by means of plasmid vectors. Preference is given to those transgenic expression vectors which enable a stable integration of the transgenic expression cassette into the host genome. In this context, host genome means the entire hereditary information of the host and comprises for example not only the chromosomal DNA of the nucleus, but also the DNA of the plastids and mitochondria. However, the insertion into the chromosomal DNA of the nucleus is preferred.

In the case of injection or electroporation of DNA into plant cells, no particular demands on the plasmid used are made. It is possible to use simple plasmids such as those of the pUC series. If complete plants are to be regenerated from the transformed cells, it is necessary for an additional selectable marker gene to be present on the plasmid.

Transformation techniques have been described for various monocotyledonous and dicotyledonous plant organisms. Furthermore, various possible plasmid vectors which normally contain a replication origin for propagation in *E. coli* and a marker gene for selection of transformed bacteria are available for introducing foreign genes into plants. Examples are pBR322, pUC series, M13 mp series, pACYC184 etc.

The transgenic expression cassette may be introduced into the vector via a suitable restriction cleavage site. The resultant plasmid is first introduced into *E. coli*. Correctly transformed *E. coli* cells are selected, cultivated and the recombinant plasmid is obtained using methods familiar to the skilled worker. Restriction analysis and sequencing may be used in order to check the cloning step.

Transformed cells, i.e. those which contain the introduced DNA integrated into the DNA of the host cell may be selected from untransformed cells, if a selectable marker is part of the introduced DNA. A marker may be, by way of example, any gene which is capable of imparting a resistance to antibiotics or herbicides. Transformed cells which express such a marker gene are capable of surviving in the presence of concentrations of an appropriate antibiotic or herbicide, which kill an untransformed wild type. Examples are the bar gene which imparts resistance to the herbicide phosphinothricin (Rathore K S et al., Plant Mol Biol. 1993 March; 21 (5):871-884), the nptII gene which imparts resistance to kanamycin, the hpt gene which imparts resistance to hygromycin and the EPSP gene which imparts resistance to the herbicide glyphosate.

Depending on the method of DNA introduction, further genes may be required on the vector plasmid. If agrobacteria are used, the transgenic expression cassette is to be integrated into specific plasmids, either into an intermediate vector (shuttle vector) or a binary vector. If, for example, a Ti or Ri plasmid is to be used for transformation, at least the right border, in most cases, however, the right and the left border, of the Ti or Ri plasmid T-DNA is connected as flanking region with the transgenic expression cassette to be introduced. Preference is given to using binary vectors. Binary vectors can replicate both in *E. coli* and in *Agrobacterium*. They normally contain a selection marker gene and a linker or polylinker flanked by the right and left T-DNA border sequences. They may be transformed directly into *Agrobacterium* (Holsters et al., Mol. Gen. Genet. 163 (1978), 181-187). The selection marker gene permits selection of transformed agrobacteria; an example is the nptII gene which imparts a resistance to kanamycin. The *Agrobacterium* which in this case acts as the host organism should already contain a plasmid with the vir region. This region is required for the transfer of T-DNA onto the plant cell. An *Agrobacterium* transformed in this way may be used for transformation of plant cells.

The use of T-DNA for transformation of plant cells has been intensely studied and described (B. Jenes et al., Techniques for Gene Transfer, in: Transgenic Plants, Vol. 1, Engineering and Utilization, edited by Kung S D and Wu R, Academic Press (1993), pp. 128-143 and in Potrykus (1991) Annu Rev Plant Physiol Plant Molec Biol 42:205-225; EP 120516; Hoekema, In: The Binary Plant Vector System, Offsetdrukkerij Kanters B. V., Alblasserdam, Chapter V; Fraley et al., Crit. Rev. Plant. Sci., 4:1-46 and An et al. (1985) EMBO J. 4:277-287). Various binary vectors are known and partly commercially available, such as, for example, pBIN19 (Bevan et al. (1984) Nucl Acids Res 12:8711f.; Clontech Laboratories, Inc. USA) or PSUN derivatives (SunGene GmbH & Co. KGaA; WO 02/00900). The expression cassette according to the invention can be inserted into these binary vectors and integrated into the plant genome as described hereinbelow.

The DNA is transferred into the plant cell by coculturing plant explants with *Agrobacterium tumefaciens* or *Agrobacterium rhizogenes.* Starting from infected plant material (e.g. leaf, root or stem parts, but also protoplasts or plant cell suspensions), it is possible to regenerate whole plants by using a suitable medium which may contain, for example, antibiotics or biocides for selection of transformed cells. The plants obtained may then be screened for the presence of the introduced DNA, in this case the transgenic expression cassette of the invention. As soon as the DNA has integrated into the host genome, the corresponding genotype is normally stable and the corresponding insertion is also found again in subsequent generations. Normally, the integrated transgenic expression cassette contains a selection marker which imparts to the transformed plant a resistance to a biocide (for example a herbicide), a metabolism inhibitor such as 2-DOG or an antibiotic such as kanamycin, G 418, bleomycin, hygromycin or phosphinothricin etc. The selection marker allows the selection of transformed cells from untransformed cells (McCormick et al. (1986) Plant Cell Reports 5:81-84). The plants obtained may be cultivated and crossed in the common manner. Two or more generations should be cultured in order to ensure that the genomic integration is stable and heritable.

As soon as a transformed plant cell has been prepared, it is possible to obtain a complete plant by using methods known to the skilled worker. To this end, callus cultures are used as starting point, by way of example. From these still undifferentiated cell masses, it is possible to induce formation of shoot and root in the known manner. The shoots obtained can be planted out and cultivated.

The integration of the T-DNA can be determined e.g. on the basis of the efficacy of expression of the nucleic acids to be expressed transgenically or of the selection marker for example in vitro by shoot meristem propagation using one of the above-described selection methods.

The invention further relates to cells, cell cultures, parts, such as, for example, roots, leaves, etc. in the case of transgenic plant organisms, and transgenic propagation material such as seeds, tubers, beets/swollen tap roots or fruits derived from the above-described transgenic organisms.

Genetically modified plants of the invention, which can be consumed by humans and animals, may also be used, for example directly or after preparation known per se, as foodstuffs or feedstuffs.

The invention further relates to the use of the above-described transgenic organisms of the invention and of the cells, cell cultures, parts, such as, for example, roots, leaves, etc., in the case of transgenic plant organisms, and transgenic propagation material such as seeds, tubers, beets/swollen tap roots or fruits derived from them for the production of food- or feedstuffs, pharmaceuticals or fine chemicals.

The invention also relates to the use of an isolated nucleic acid of the invention, an expression cassette according to the invention or a vector of the invention for the manufacturing of a transgenic plant that is resistant to clubroot disease and/or infection with the pathogen *Plasmodiophora brassicae.*

The present invention further relates to a method for the manufacturing of a transgenic plant that is resistant to clubroot disease, comprising the following steps:

a) introducing into a plant cell an isolated nucleic acid of the invention, an expression cassette of the invention or a vector of the invention; and

b) selection of transgenic cells which comprise said isolated nucleic acid, expression cassette or vector stably integrated into the genome; and

c) regeneration of intact plants from said transgenic cells.

Information on how these steps may be performed are given in detail above.

In the following the present invention is further described by way of examples.

**Figures:**

FIG. 1 shows that *PYK10:CKX3-GFP* (shortened as *P10:CKX3)* expression in *Arabidopsis* does not significantly influence shoot development. In (A) rosette diameter and in (B) rosette fresh weight in P10:CKX3 and control plants grown in hydroponic culture for 34 days are shown. Independent *P10:CKX3* homozygous lines 1-1, 10-1 and 16-5 were analysed and compared to wildtype Col-0 and to a homozygous line 35S:CKX1 which expresses CKX1 under the control of the systemic promoter 35S. Data represent mean values ± SD ($n \geq 20$). Student's t test was used to compare values to the wild type. *, $P < 0.05$. WT, wild type.

## Examples:

### Example 1: Expression of CKX genes under the control of a promoter which is predominantly active in the hypocotyls does not adversely affect shoot development

#### *Plasmid construction*

The 35S promoter of plasmid pBinSMGFP (Werner et al., 2003) was cut out with *Eco*RI and *Kpn*I and replaced by a synthetic double-stranded oligonucleotide (link-plus (SEQ ID No. 25) 5'-CGGAATTCCTAGGCTTCTGCCCGGGCTTCTGGGTACCCC-3' and link-minus (SEQ ID No. 26) 5'-GGGGTACCCAGAAGCCCGGGCAGAAGCCTAGGAATTCCG-3') containing *Eco*RI-*Avr*II-*Sma*I-*Kpn*I sites. A *AtCKX3* genomic fragment was subcloned via *Kpn*I/*Xho*I restriction sites from pBS-AtCKX3 (Werner et al., 2001) to generate a N-terminal fusion with GFP. Next, a 1456 bp promoter fragment of the *PYK10* gene (At3g09260) was amplified by PCR from DNA of *Arabidopsis thaliana* Columbia (Col-0) using the forward primer (SEQ ID No. 27) 5'-GACCCGGGACTGCAACGAAGTGTA-3' and the reverse primer (SEQ ID No. 28) 5'-CAGGTACCTTTTTGTTTGTAATTCTG-3'. The fragment was first cloned into pCR2.1-TOPO (Invitrogen) and further subcloned in *Sma*I/*Kpn*I sites upstream of the *CKX3-GFP* fusion gene, resulting in vector pBinP10:CKX3-GFP.

#### *Transgenic Plants and Growth Conditions*

*Arabidopsis thaliana* accession Col-0 was used for transformation by the floral dip method (Clough and Bent, 1998) and 40 independent *P10:CKX3* transgenic lines were selected after surface sterilization of seeds on half-strength MS medium (Murashige and Skoog, 1962) containing 15 mg/L hygromycin. Plants were grown in a greenhouse under 16 h light/8 h dark cycles at 21 °C (light period) and 18°C (dark period). *In vitro* experiments were carried out on half-strength MS medium supplemented with 1 % sucrose. The hydroponic system used was basically as described previously (Tocquin et al., 2003). 0.7 ml microfuge tubes filled with 1/4 MS medium, 0.8% agar, with their conical ends cut off were used as seed holders. *Arabidopsis* plants were cultured in low-strength modified Hoagland nutrient solution containing 1.25 mM $KNO_3$, 1.5 mM $Ca(NO_3)_2$, 0.75 mM $MgSO_4$, 0.28 mM $KH_2PO_4$, 50 μM KCl, 25 μM $H_3BO_3$, 5 μM $MnSO_4$, 1.0 μM $ZnSO_4$, 0.5 μM $CuSO_4$, 0.1 μM $Na_2MoO_4$, 25 μM FeNaEDTA, and 3 mM MES, pH 5.7. Rosette size was determined using a ruler.

As described in the literature (see e.g. Werner et al. 2001 and Werner et al. 2003) systemic overexpression of CKX1 or CKX3 leads to virtually the same consequences for the transgenic plant. Thus systemic overexpression of CKX1 can serve as suitable comparative example to CKX3 expression under the control of a promoter predominantly active in the hypocotyls. As can be seen in FIG. 1 A and B, whereas systemic expression of CKX1 (35S:CKX1) yields a significant growth retardation in comparison to wild type Col-0 plants, rosette growth of plants expressing CKX3 under a promoter predominantly active in the hypocotyls show a growth comparable to wild type Col-0.

### Example 2: Expression of a CKX gene under the control of a promoter which is predominantly active in the hypocotyl confers resistance to clubroot

#### Phytopathological analysis:

Cultivation and inoculation conditions of *A. thaliana* plants were performed according to Siemens et al. (2002) (Siemens, J., Nagel, M., Ludwig-Müller, J. & Sacristán, M. D. (2002) The interaction of Plasmodiophora brassicae and Arabidopsis thaliana: Parameters for disease quantification and screening of mutant lines. Journal of Phytopathology, 150, 592-605). Fourteen-day-old plants cultivated under controlled environment (21 °C, 16 h light, 100 μmol photons $s^{-1}$ $m^{-2}$) were inoculated by injecting the soil around each plant with 2 ml of a resting spore suspension of the pathogen with a standard concentration of $10^6$ to $10^7$ spores/ml. Disease symptoms were assessed 28 days after inoculation (dpi) using a scale consisting of five classes according to Siemens et al. (2002). Disease index (DI) was calculated using the five-grade scale according to the formula: DI = $(1n_1+2n_2+3n_3+4n_4)$ $100/4N_t$, where $n_1$ to $n_4$ is the number of plants in the indicated class and $N_t$ total number of plants tested.

For quantitative estimation the plants were cut at the top of the hypocotyl into shoots and roots. The infected roots were measured as galls, ignoring occasionally remaining non-infected lateral roots. The leaves and bolting stalks were measured as shoots, ignoring possible slight gall formation in rosette leaves. After determination of shoot and root fresh weight, the root index $R_i/R_{ni}$ (roots of infected plants/roots of not infected plants) was calculated.

As pathogen isolates of *Plasmodiophora brassicae* single spore isolates ($e_3$) or field isolates (R and S) were used. The single spore isolates have been described by Fähling et al. (Fähling, M., Graf, H. & Siemens, J. (2003) Pathotype-separation of Plasmodiophora brassicae by the host plant. Journal of Phytopathology 151, 425-430.). Field isolates R and S were originally collected by the Federal Institute of Plant Breeding (Quedlinburg). These isolates have the ECD code (16/31/31) and (16/18/12), respectively. These field isolates are aggressive to all or some lines of the crop plant *Brassica napus.*

**Clubroot resistance caused by locally enhanced cytokinin breakdown**

Hypocotyl and root-specific expression of *CKX3* in transgenic lines (P10:CKX3, lines 10-1 and 1-1) of the susceptible ecotype Col-0 revealed quantitative resistance to infection by *P. brassicae*. The galls of *P10:AtCKX3* lines were smaller compared to galls of Col-0. The disease indices of P10:CKX3 lines were significantly lower than the disease indices of Col-0 (Table 2). When roots and hypocotyl symptoms were separately scored at 21 dpi, the root symptoms of the P10:CKX3 lines showed a stronger resistance (tolerance).
To explore whether the resistance of P10:CKX3 is narrow (i.e. race-specific) or of a broader type two additional field isolates of *P. brassicae* with high virulence to Brassica crop plants were tested (the above-mentioned 'R' and 'S' isolates). The same effect of increased tolerance was noted in P10:CKX3 lines (Table 3). Infection with both field isolates revealed hypertrophy and hyperplasia in lateral and main roots but the hypocotyl was only partial thickened. P10:CKX3 lines also showed tolerance to both field isolates with small galls at the roots but no thickening at the hypocotyl. The shape of galls is taken as an indication of slower spreading in the host. Necrosis at the root surface was only observed with the isolate $e_3$.
Histological analysis reveals the reduction of hypertrophy and hyperplasia within the root and hypocotyl of both P10:CKX3 lines for all isolates (data not shown). Correspondingly the vegetative plasmodia are smaller and in younger developmental stages than in wild type plants (Col-0).

**Table 2:** Resistance of *P10:CKX3* transgenic plants (line 10-1 and line 1-1) against infection with isolate $e_3$ of *P. brassicae.* Different inoculation pressures were used as indicated. The number of plants, the disease indices (DI) and the root index ($R_i/R_{ni}$) of lines are given.

| line | Inoculum (spores/ml) | Disease index | No of plants | $R_i/R_{ni}$ |
|---|---|---|---|---|
| Col | $5x10^6$ | 99,4 | 40 | 3,56 |
| Col | $10^7$ | 100 | 38 | 3,22 |
| 10-1 | $5x10^6$ | 66,8 | 37 | 1,85 |
| 10-1 | $10^7$ | 66,4 | 35 | 1,73 |
| 1-1 | $5x10^6$ | 69,5 | 46 | 1,81 |
| 1-1 | $10^7$ | 71,3 | 48 | 1,78 |

**Table 3:** Resistance of *P10:CKX3* transgenic plants (line 10-1 and line 1-1) against different isolates ($e_3$, R, S) of *P. brassicae.* Inoculation pressure was $5x10^6$ spores/ml. The number of plants, the disease indices (DI) and the root index ($R_i/R_{ni}$) of lines are given.

| line | isolate | Disease index | No of plants | $R_i/R_{ni}$ |
|---|---|---|---|---|
| Col | $e_3$ | 99.0 | 48 | 3.88 |
| 10-1 | $e_3$ | 40.0 | 90 | 1.72 |
| 1-1 | $e_3$ | 51.7 | 88 | 1.74 |
| Col | R | 70.8 | 72 | 2.99 |
| 10-1 | R | 42.2 | 92 | 1.68 |
| 1-1 | R | 45.3 | 96 | 1.64 |
| Col | S | 51.5 | 68 | 2.54 |
| 10-1 | S | 29.1 | 98 | 1.31 |
| 1-1 | S | 31.3 | 88 | 1.21 |

## Claims

**1.** An isolated nucleic acid, comprising

a) at least one promoter sequence which is predominantly active in the hypocotyls compared to shoot apex tissue of a plant; and
b) a nucleic acid sequence encoding for a protein that is able to decrease a cytokinin status in a plant tissue;

wherein the nucleic acid sequence encoding said protein is located in 3'-position to the at least one promoter and wherein the at least one promoter sequence and the nucleic acid sequence encoding said protein are functionally linked with one another.

**2.** An isolated nucleic acid of claim 1, wherein the at least one promoter sequence additionally is predominantly active in root tissue compared to shoot apex tissue of a plant.

**3.** An isolated nucleic acid of one of the preceding claims, wherein the at least one promoter sequence is selected from

i) SEQ ID Nos. 1, 2, 3, 4, 5, 12, 13, 14, 15, 16, 17, 18, 19, 20, 21, 22, 23 or 24 or a reverse complement thereof; or
ii) from functionally equivalent sequences which have at least 60% homology with one of the sequence of i) over a sequence segment of at least 100 base pairs and which function as promoter sequence which is predominantly active in the hypocotyls compared to shoot apex tissue of a plant; or
iii) from functionally equivalent fragments of one of the promoter sequences of i) or ii) with a length of at least 100 base pairs and which function as promoter sequence which is predominantly active in the hypocotyls compared to shoot apex tissue of a plant.

**4.** An isolated nucleic acid of one of the preceding claims, wherein the isolated nucleic acid comprises a nucleic acid sequence encoding for a protein with cytokinin oxidase/dehydrogenase activity.

**5.** An isolated nucleic acid of claim 4, wherein the nucleic acid sequence encoding for a protein that is able to decrease a cytokinin status in a plant tissue is selected from

i) one of SEQ ID Nos 6, 7, 8, 9, 10, 11 or 29; or
ii) from sequences which have at least 60% homology with one of the sequence of SEQ ID Nos. 6, 7, 8, 9, 10, 11 or 29 over a coding sequence segment of at least 300 base pairs and which encode for a protein with cytokinin oxidase/dehydrogenase activity; or
iii) from sequences which hybridise under standard conditions with one of the nucleic acid sequences described in SEQ ID Nos. 6, 7, 8, 9, 10, 11 or 29 or with a nucleic acid sequences complementary thereto, and which encode for a protein with cytokinin oxidase/dehydrogenase activity.

**6.** A transgenic expression cassette for the expression of nucleic acids comprising an isolated nucleic acid of one of claims 1 to 5.

**7.** A vector comprising an isolated nucleic acid of one of claims 1 to 5 or a transgenic expression cassette of claim 6.

**8.** A transgenic organism transiently or stably transformed or transfected with an isolated nucleic acid of claims 1 to 5, an expression cassette of claim 6 or a vector of claim 7.

**9.** A transgenic organism of claim 8, wherein the organism is a plant or a microorganism.

**10.** A transgenic microorganism of claim 9, wherein the plant is selected from the family Brassicaceae, preferably from the genera Brassica or Arabidopsis.

**11.** A cell, cell culture, part, organ, tissue or transgenic propagation material comprising an isolated nucleic acid of claim 1 to 5, a transgenic expression cassette of claim 6 or a vector of claim 7.

**12.** Use of an isolated nucleic acid of claim 1 to 5, a transgenic expression cassette of claim 6 or a vector of claim 7 for the manufacturing of a transgenic plant that is resistant to clubroot disease or infection.

**13.** Method for the manufacturing of a transgenic plant that is resistant to clubroot disease, comprising the following steps:

a) introducing into plant cells an isolated nucleic acid of claim 1 to 5, a transgenic expression cassette of claim 6 or a vector of claim 7; and
b) selection of transgenic cells which comprise said isolated nucleic acid, expression cassette or vector stably integrated into the genome; and
c) regeneration of intact plants from said transgenic cells.

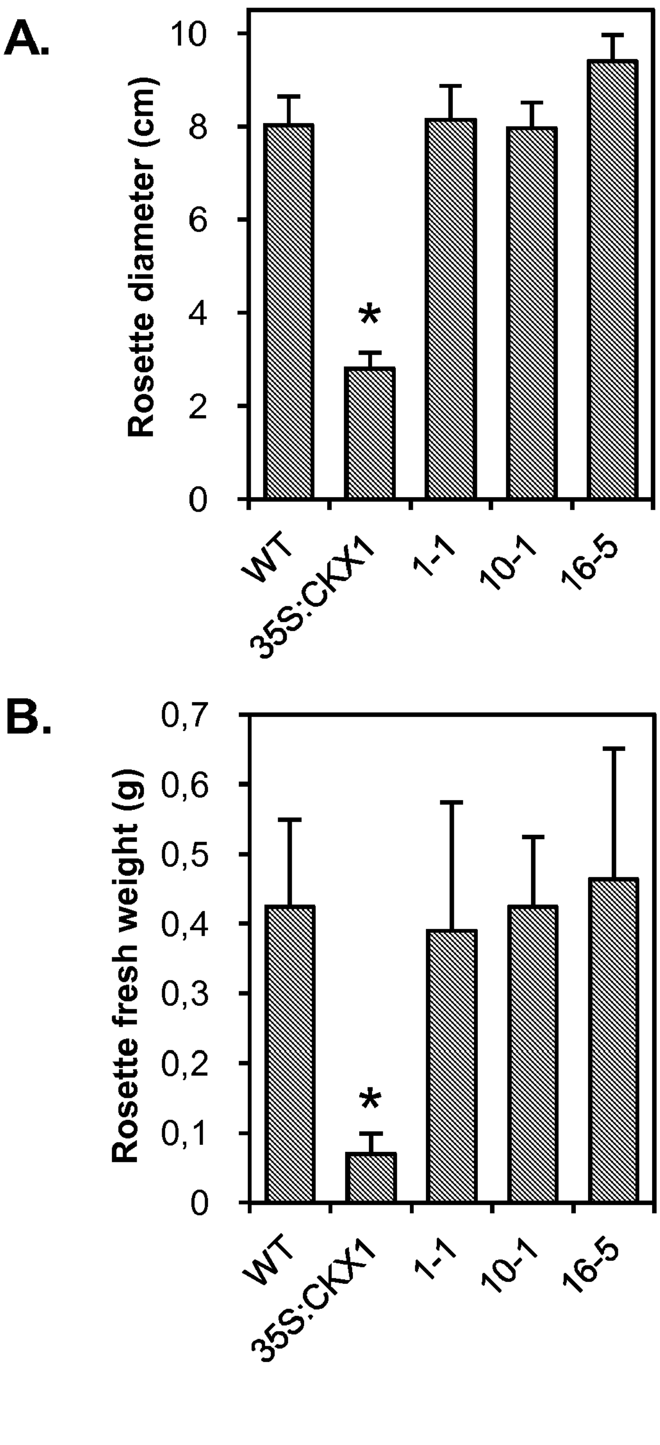
A.
10
8
6
4
2
0
Rosette diameter (cm)
*
WT
35S:CKX1
1-1
10-1
16-5
B.
0,7
0,6
0,5
0,4
0,3
0,2
0,1
0
Rosette fresh weight (g)
*
WT
35S:CKX1
1-1
10-1
16-5

FIG. 1

Europäisches Patentamt
European Patent Office
Office européen des brevets

# EUROPEAN SEARCH REPORT

Application Number
EP 09 16 5807

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WERNER T ET AL: "Cytokinin-deficient transgenic Arabidopsis plants show multiple developmental alterations indicating opposite functions of cytokinins in the regulation of shoot and root meristem activity" PLANT CELL, AMERICAN SOCIETY OF PLANT PHYSIOLOGISTS, ROCKVILLE, MD, US, vol. 15, no. 11, 1 November 2003 (2003-11-01), pages 2532-2550, XP002288482 ISSN: 1040-4651 * abstract; pages 2542 and 2546 * ----- | 1-2,4-11 | INV. C12N15/82<br>ADD. C12N15/53 |
| X | Sarx, Johannes: "Die Bedeutung der Cytokinin-Oxidase bei der Infektion von Arabidopsis thaliana mit dem Pathogen Plasmodiophora brassicae"<br>July 2003 (2003-07), XP002557940 Retrieved from the Internet: URL:http://tu-dresden.de/die_tu_dresden/fakultaeten/fakultaet_mathematik_und_naturwissenschaften/fachrichtung_biologie/biotechnologen/Wirt-Parasit-Interaktion/Johannes_Sarx.pdf> [retrieved on 2009-11-26] * pages 1, 2, 9-11, 61-63, 67, 68, 72; Table 4.2 * ----- | 1-13 | TECHNICAL FIELDS SEARCHED (IPC)<br>C12N |

-/--

The present search report has been drawn up for all claims

| Place of search | Date of completion of the search | Examiner |
|---|---|---|
| Munich | 30 November 2009 | Kurz, Birgit |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 16 5807

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | SIEMENS JOHANNES ET AL: "Transcriptome analysis of Arabidopsis clubroots indicate a key role for cytokinins in disease development" MOLECULAR PLANT-MICROBE INTERACTIONS, vol. 19, no. 5, May 2006 (2006-05), pages 480-494, XP002557941 ISSN: 0894-0282 * abstract; pages 480, 484-487 and 490 * ----- | 1-13 | |
| A | CSUKASI FABIANO ET AL: "Modification of plant hormone levels and signaling as a tool in plant biotechnology" BIOTECHNOLOGY JOURNAL, vol. 4, no. 9, 7 July 2009 (2009-07-07), pages 1293-1304, XP002557942 ISSN: 1860-6768 * abstract; pages 1293 and 1299-1301 * ----- | 1-13 | |
| A | NITZ I. ET AL.: "PYK 10, A SEEDLING AND ROOT SPECIFIC GENE AND PROMOTER FROM ARABIDOPSIS THALIANA" PLANT SCIENCE, ELSEVIER IRELAND LTD, IE, vol. 161, no. 2, 1 January 2001 (2001-01-01), pages 337-346, XP001013257 ISSN: 0168-9452 * abstract; pages 337, 338, 341, 341 and 345; Figure 5 * -/-- | 1-13 | TECHNICAL FIELDS SEARCHED (IPC) |

The present search report has been drawn up for all claims

1

| Place of search | Date of completion of the search | Examiner |
|---|---|---|
| Munich | 30 November 2009 | Kurz, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 16 5807

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | -& DATABASE EMBL [Online] 5 January 2001 (2001-01-05), Nitz, I.: "Arabidopsis thaliana partial pyk10 gene for myrosinase, promoter region" XP002557943 retrieved from EBI accession no. EMBL:AJ292756 Database accession no. AJ292756 * the whole document * | 1-13 | |
| A | WO 01/44454 A2 (DAS REKTORAT DER CHRISTIAN ALB [DE]; GRUNDLER FLORIAN [DE]; NITZ INKE) 21 June 2001 (2001-06-21) * abstract; pages 1, 5, 6 and 11; claims 1-11 * | 1-13 | |
| A | WO 01/90314 A1 (UNIV NEW YORK [US]; BENFEY PHILIP N [US]; HELARIUTTA YRJO [FI]; FUKAKI) 29 November 2001 (2001-11-29) * claim 6 * | 1-13 | TECHNICAL FIELDS SEARCHED (IPC) |
| A | LUDWIG-MÜLLER, JUTTA: "Kohlhernie-neue Ansätze zur Bekämpfung der gefürchteten Kohlerkrankung" BIOLOGIE IN UNSERER ZEIT, vol. 30, no. 1, 11 February 2000 (2000-02-11), pages 14-23, XP002557944 * pages 15 and 17-20 * | 1-13 | |

The present search report has been drawn up for all claims

1

| Place of search | Date of completion of the search | Examiner |
|---|---|---|
| Munich | 30 November 2009 | Kurz, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 09 16 5807

This annex lists the patent family members relating to the patent documents cited in the above-mentioned European search report.
The members are as contained in the European Patent Office EDP file on
The European Patent Office is in no way liable for these particulars which are merely given for the purpose of information.

30-11-2009

| Patent document cited in search report | Publication date | Patent family member(s) | Publication date |
|---|---|---|---|
| WO 0144454 A2 | 21-06-2001 | CA 2396539 A1 | 21-06-2001 |
| | | CN 1434869 A | 06-08-2003 |
| | | DE 19960843 A1 | 28-06-2001 |
| | | EP 1244802 A2 | 02-10-2002 |
| | | JP 2003519475 T | 24-06-2003 |
| | | US 2003177536 A1 | 18-09-2003 |
| WO 0190314 A1 | 29-11-2001 | AU 6509601 A | 03-12-2001 |
| | | CA 2409949 A1 | 29-11-2001 |
| | | EP 1290142 A1 | 12-03-2003 |
| | | US 6927320 B1 | 09-08-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4237224 A
- EP 2007008331 W
- US 5565350 A
- WO 0015815 A
- US 6110736 A
- WO 9845456 A
- US 5489520 A
- US 5510471 A
- US 5776760 A
- US 5864425 A
- US 5633435 A
- US 5627061 A
- US 5463175 A
- EP 0218571 A
- WO 9927116 A
- EP 0807836 A
- EP 0601092 A
- WO 9741228 A
- EP 120516 A
- WO 0200900 A

### Non-patent literature cited in the description


- **Siemens et al.** *Journal of Phytopathology,* 2002, vol. 150, 592-605
- **Siemens et al.** *Molecular Plant-Microbe Interactions,* 2006, vol. 19, 480-494
- **Higo K et al.** *Nucl Acids Res,* 1999, vol. 27 (1), 297-300
- **Schenborn E ; Groskreutz D.** *Mol Biotechnol,* 1999, vol. 13 (1), 29-44
- **Chui W L et al.** *Gurr Biol,* 1996, vol. 6, 325-330
- **Leffel S M et al.** *Biotechniques,* 1997, vol. 23 (5), 912-8
- **Millar et al.** *Plant Mol Biol Rep,* 1992, vol. 10, 324-414
- **Jefferson et al.** *EMBO J.,* 1987, vol. 6, 3901-3907
- **Sambrook J ; Fritsch E F ; Maniatis T et al.** Molecular Cloning-A Laboratory Manual. Cold Spring Harbor Laboratory Press, 1989, 9.31-9.57
- Current Protocols in Molecular Biology. John Wiley & Sons, 1989, 6.3.1-6.3.6
- **Kunkel et al.** *Methods Enzymol,* 1987, vol. 154, 367-382
- **Tomic et al.** *Nucl Acids Res,* 1990, vol. 12, 1656
- **Upender et al.** *Biotechniques,* 1995, vol. 18 (1), 29-30
- **Maniatis T et al.** Molecular Cloning: A Laboratory Manual. Cold Spring Harbor Laboratory, 1989
- **Silhavy T J et al.** Experiments with Gene Fusions. Cold Spring Harbor Laboratory, 1984
- **Ausubel F M et al.** Current Protocols in Molecular Biology. Greene Publishing Assoc. and Wiley Interscience, 1987
- **Lam E ; Chua N H.** *J Biol Chem,* 1991, vol. 266 (26), 17131-17135
- **Schöffl F et al.** *Mol Gen Genet,* 1989, vol. 217 (2-3), 246-53
- **Rouster J et al.** *Plant J,* 1998, vol. 15, 435-440
- **Lohmer S et al.** *Plant Cell,* 1993, vol. 5, 65-73
- **Sauer B.** *Methods (Duluth),* 1998, vol. 14 (4), 381-92
- **Ebinuma H et al.** *Proc Natl Acad Sci USA,* 2000, vol. 94, 2117-2121
- **McCormick et al.** *Plant Cell Reports,* 1986, vol. 5, 81-84
- **de Block et al.** *EMBO J.,* 1987, vol. 6, 2513-2518
- **Vikkers JE et al.** *Plant Mol Biol Reporter,* 1996, vol. 14, 363-368
- **Thompson CJ et al.** *EMBO J.,* 1987, vol. 6, 2519-2523
- **Steinrucken H C et al.** *Biochem Biophys Res Commun,* 1980, vol. 94, 1207-1212
- **Levin J G ; Sprinson D B.** *J Biol Chem,* 1964, vol. 239, 1142-1150
- Mode of action of glyphosate; A literature analysis. **Cole D J.** The herbicide glyphosate. Buttersworths, 1985, 48-74
- New weed control opportunities: development of soybeans with a Roundup Ready gene. **Padgette S R et al.** Herbicide Resistant Crops. CRC Press, 1996, 53-84
- **Saroha M K ; Malik V S.** *J Plant Biochemistry and Biotechnology,* 1998, vol. 7, 65-72
- **Padgette S R et al.** *Crop Science,* 1995, vol. 35 (5), 145-1461
- **Padgette S R et al.** *J Nutr,* 1996, vol. 126 (3), 702-16

- **Shah D et al.** *Science,* 1986, vol. 233, 478-481
- **Beck et al.** *Gene,* 1982, vol. 19, 327-336
- **Randez-Gil et al.** *Yeast,* 1995, vol. 11, 1233-1240
- **Sanz et al.** *Yeast,* 1994, vol. 10, 1195-1202
- **Sathasivan K et al.** *Nucleic Acids Res.,* 1990, vol. 18 (8), 2188
- **Datta N ; Richmond M H.** *Biochem J,* 1966, vol. 98 (1), 204-9
- **Heffron F et al.** *J. Bacteriol,* 1975, vol. 122, 250-256
- **Bolivar F et al.** *Gene,* 1977, vol. 2, 95-114
- Selection of marker-free transgenic plants using the oncogenes (ipt, rol A, B, C) of Agrobacterium as selectable markers. **Ebinuma H et al.** Molecular Biology of Woody Plants. Kluwer Academic Publishers, 2000
- **Koprek T et al.** *The Plant Journal,* 1999, vol. 19 (6), 719-726
- **Gleave A P et al.** *Plant Mol Biol,* 1999, vol. 40 (2), 223-35
- **Perera R J et al.** *Plant Mol Biol,* 1993, vol. 23 (4), 793-799
- **Stougaard J.** *Plant J,* 1993, vol. 3, 755-761
- **Koprek et al.** *Plant J,* 1999, vol. 16, 719-726
- **Naested H.** *Plant J,* 1999, vol. 18, 571-576
- **Sundaresan V et al.** *Genes & Development,* 1995, vol. 9, 1797-1810
- **Fedoroff N V ; Smith D L.** *Plant J,* 1993, vol. 3, 273-289
- **Schenbron E ; Groskreutz D.** *Mol Biotechnol.,* 1999, vol. 13 (1), 29-44
- **Chui W L et al.** *Curr Biol,* 1996, vol. 6, 325-330
- **Sheen et al.** *Plant J,* 1995, vol. 8 (5), 777-784
- **Haseloff et al.** *Proc Natl Acad Sci USA,* 1997, vol. 94 (6), 2122-2127
- **Reichel et al.** *Proc Natl Acad Sci USA,* 1996, vol. 93 (12), 5888-5893
- **Tian et al.** *Plant Cell Rep,* 1997, vol. 16, 267-271
- **Fromm et al.** *Proc Natl Acad Sci USA,* 1985, vol. 82, 5824-5828
- **Ow et al.** *Science,* 1986, vol. 234, 856-859
- **Dellaporta et al.** *Chromosome Structure and Function: Impact of New Concepts, 18th Stadler Genetics Symposium,* 1988, vol. 11, 263-282
- **Katz et al.** *J Gen Microbiol,* 1983, vol. 129, 2703-2714
- **Prasher et al.** *Biochem Biophys Res Commun,* 1985, vol. 126 (3), 1259-1268
- **Sambrook et al.** Molecular Cloning. A Laboratory Manual. Cold Spring Harbor Laboratory Press, 1989
- **Keown et al.** *Methods in Enzymology,* 1990, vol. 185, 527-537
- **Rathore K S et al.** *Plant Mol Biol.,* March 1993, vol. 21 (5), 871-884
- **Holsters et al.** *Mol. Gen. Genet.,* 1978, vol. 163, 181-187
- Techniques for Gene Transfer. **B. Jenes et al.** Transgenic Plants, Vol. 1, Engineering and Utilization. Academic Press, 1993, vol. 1, 128-143
- **Potrykus.** *Annu Rev Plant Physiol Plant Molec Biol,* 1991, vol. 42, 205-225
- **Hoekema.** The Binary Plant Vector System. Offsetdrukkerij Kanters B. V,
- **Fraley et al.** *Crit. Rev. Plant. Sci.,* vol. 4, 1-46
- **An et al.** *EMBO J.,* 1985, vol. 4, 277-287
- **Bevan et al.** *Nucl Acids Res,* 1984, vol. 12, 8711f
- **Siemens, J. ; Nagel, M. ; Ludwig-Müller, J. ; Sacristán, M. D.** The interaction of Plasmodiophora brassicae and Arabidopsis thaliana: Parameters for disease quantification and screening of mutant lines. *Journal of Phytopathology,* 2002, vol. 150, 592-605
- **Fähling, M. ; Graf, H. ; Siemens, J.** Pathotype-separation of Plasmodiophora brassicae by the host plant. *Journal of Phytopathology,* 2003, vol. 151, 425-430

SEQUENCE LISTING

<110> Institut für Biologie/anewandte Genetik

<120> Means and method for the production of transgenic plants that are resistant to clubroot

<130> P629909EP

<160> 29

<170> PatentIn version 3.3

<210> 1
<211> 1457
<212> DNA
<213> Arabidopsis thaliana

<400> 1

```
actgcaacga agtgtaccaa caacttgact aggattctaa gttcttttat gtataggatg      60
tctatattaa actaccatga ctaacatata tatagtagtt ccatatgctc gataaactat     120
gatagatcaa caattttaaa catatagttt aacactattt atttgttcaa cgtcaatagt     180
ttatagttac gcatgcgctc ggcttagatt tggtccccaa cagtcgaaat tgtcaaataa     240
tataaaataa aagtttcatt gttaggattc atttattctt cgggtggtta ttgtaataaa     300
aggcaaaaga aaaagaagaa caaaattcac aagtaaaaaa aaagataaca tcattctttt     360
agtcgacaaa aaaaaaaaaa aatcaaaaag atttattcag tactacagtt taatattgtt     420
ttgacttttt tctttttctt tatattatct gaaaattcta gactgcagct gaaacatgtg     480
atatggatta aaggcgtatc cagtatccac ataaagagga gtggtgtcgc tcacccagtc     540
acccttgtta cttgttagat agcattaata catttgtaag caacagctta tctgatagac     600
atgtcttaat tgggaaatat gctctaagat gatacaacca tggttccaac tgttgaccac     660
catagctgat aacatgttga ttacattttt tcttttcagt tataacgatt acttttttgg     720
ggaaattatt gatataatat gattcattgg atgatccgat atcatgcata taaagttgta     780
tctcgtgaaa cacgagatag tattatactc cattctttca ttatcggagt atgtttaaaa     840
tttgaaaaca aatacagaca cggaccgtgg tctttacctt cagaaaaaaa aagagaaaaa     900
aaaaacaatc cactgtttat tataggagtt gtagaaaatc gggcaacgat attcgatatg     960
agttattatt agggccttat tattatatgg tattactgga tattactaaa taaaataatc    1020
atataaattt cacattttaa tatacactcg ttggacacgc ggaatattat atgttctaaa    1080
tgttaaaaaa atcaaaagaa atacaacgat cgacggatct agagtctaga ccatgcaaat    1140
aaatcatcct atttaaatat aataactgtg catatagttt agtcaaataa aaaggtaaag    1200
aaacaatata caacctataa cgtcaatatc catgtacgta gtaataatta ggatatgaca    1260
gaaaacacga tatcttgata tatacaaaat gaaaacttaa aaattgatta atatggcctg    1320
gctgggtata ttattataaa aacataaaga gagatcaata attgattcga agatcactat    1380
ataaagaacg tcttcgatat gtaaaagaac catcctaaac attttttctt gaataaaatc    1440
agaattacaa acaaaaa                                                   1457
```

<210> 2
<211> 1384
<212> DNA
<213> Arabidopsis thaliana

<400> 2
aacttcggaa aagtcaacgt acgatgacgt tttcacttgc gtcactctca tgatttcatt 60
tattcttgta taatataaag gtagcggtag tgtgcaaata tcaaataagt agtttaatta 120
gtaccaatca ttttattcat tattttttt agtagaatat ttggatgttg aaaatataaa 180
tttaattttg tatttgttga tgttataaat ttattgattg tataaacatt cttagtcatc 240
agtttcgtta agtccatatc taaacacttc atatctgcta aatagtcaat agattataaa 300
attggatcag gaaaaagtaa atcggagcta taaaaaaata gtgtgcaacg aaaagacaat 360
taattagtta aaaatacata caaatctaaa caaattcaaa atttcaatag tggaaaacac 420
caatcaaatg gataatgctg tcgaagatta tctacaaagc atcaataaaa gtaaataatt 480
aatattatct tacatgctat tataaaagat tatgagatta ggagtataat tgtcaagcaa 540
ctgagcaaga gggtaaggtt tggttattat atatgtggag ccctatacga agttatgtag 600
aaacaaagaa atatcaagtt gcttcaaatc atatcctagc aagacaaccc tacaagacaa 660
gcaatttgat gaatttgtct ctccttttta ttcgagtgaa agtcattatc ttcttatctt 720
tttactcgaa tgtgaatatg caaaatatct tttgatattt aagagcttac ctagtgagtc 780
attactacta cgaaaatcat atatcaatct tatcataaaa cttttaagat aaaaaaaaaa 840
aagaaaaaac ttttgagaga ttggctttta aagacttaag ttacgattat aaacactagt 900
agttcaagtc tttttggttt tggtttgtgt tatgttttag atttaaaatt tcaaatgaac 960
ctacgtcctt aaccaactca atcaaaattc tagttaaaaa aaataatcac cattttgtta 1020
gcattcagct taggattcga accatgggta gctcaaggta ttttaaactc tagaggaata 1080
aaatggatgt tagtgaaatt tgtcagcatc atagacaaga tcaagttggc acaacttgaa 1140
gggtcctgac aaaatatctt aagttgcctc cataaatgtt taatggataa gacttggccc 1200
cacagagtta aaccagagag acacagagag agacttttga cacctcaccc atggctgcgt 1260
taacacatgt ttaggattcc tttctttata tagccaacaa tatcatcaaa actttttctt 1320
caaacaccac ttgcagtttt tcttattctc ctgtcttgtc taaagaaaaa agagagagga 1380
agaa 1384

<210> 3
<211> 2000
<212> DNA
<213> Arabidopsis thaliana

<400> 3
agcatgggtg gaccgtggac cggttattgt ttgctagccg aacaaaaagg actaaatatg 60
acactatatg ttttgggggt ctagacaggc ccattattcg cattttgggc cttgacaata 120
catctaacat ggttgatggt acacatccct tgtgaggatc caaattcact agtctcttaa 180

caaatagtat ttttcataac aaatcttaat aattttttac gaagaggagg taatattatg 240
ccaatatttc tcttttaagt aacatattta tttccaaacg tacaattaaa tgaaaaacgt 300
gtttagtgga actacaacaa tatcataaat caacatgact tgtttaaaat ggagtaaaaa 360
ataaaaaaat gtcatgattt tgctttgaaa tagacaatga aattttgtgt ttaataaata 420
aatagaccat caaaattgat gcttatacgt aacaaagtat taacgcatag aattaatcga 480
taagcaccaa ttaccaaata ctaaactagc tagtctactc tataatcccg aattgtaact 540
tcattcatac tattaaaaaa tattttcgga gaaaaagtca aaagaaatgg ttcaatcaaa 600
tgtctccaca ttattttgtt aagcaggttg ctgaatataa aataattcga aatctctact 660
atattttatc atcctgtctg tctctggtac tacagaccag atgttgagta caaacacgta 720
ccatgaggca tcgcggtaat gcatcaagtg tcacttagac tgttcgacac atgtttgagg 780
attgcatcaa aaaggtggct acgaagtcta cagcttctag gaagagtcca agcggaccaa 840
ttaggatttg ttctatgtat ggtcaaaacc tatatagaat ttgtttgcta gcgttgctat 900
tatctttttt tctttttgtt gattaaaaaa aactatgttc tccgaatgta aaaaaaaaac 960
aacaacgaag ttctgctcca gatactgttt taaccggcca attcagaata aactgattta 1020
aatacatgta cccattcttg aaggtcaaac gacaaaaaat atatacaaat tgtttgctag 1080
tgaattacta ttatcttttt tcgtgtattt ttttgttgat tagaaactat gttctacgaa 1140
tgtaaagaaa aaatacaaca acgaagttgt gctccagata ctgttctgac cgaccaattc 1200
tgaattacct gagtagtcca ttttaaggca tgtacccatt ttttctcttt tgggttaagg 1260
gtttaagttt ttgctttaaa aagaagaaat gttttgtagc catatattaa tataaaagtg 1320
tatattttat aaatatctct aacttatata ttataaagaa aaatacctaa gaaatttact 1380
agtcagaaat taatggaggc cttcgaattg cagggaattt gtcaatcaac tcttggagta 1440
cttctggttg gttttatttt agccttaacc aatccggttt agacccaagc caaatgtcaa 1500
gatgagacaa cagccacatc aacattaaaa ccaaacttaa cccaattaca atttgccatt 1560
tgcatcactc tttgttttta tatttgtaaa acacaaaagg tgggtcctaa tactaacaat 1620
gtgaaaacca taccgtacag tcctcaatta cgaagttcca gaagtttcga ctattcgaat 1680
gtgcatcaaa aaggtccaaa accgtttcca gaagtttcta atttctaact tgagtgtact 1740
ataataataa tttttacaaa tattttaatc aaatctaata tcatcactca ctttctactc 1800
ttcctatata aagccacctc aaaattatct tacgtggaca ttaccttcct cactccatca 1860
actgccataa acgaacttaa tccgaccagt ttttcaaaat ccgaagatga tgaactcaac 1920
ttcggagact ggttaatcga aatcatcaag aatcttgtct ttaatttcca attgggtttt 1980
tcaaaacccg ttacctcagg 2000

<210> 4
<211> 2001
<212> DNA
<213> Arabidopsis thaliana

<400> 4
ttcgatcgtt agatattata tatcaaaatc gactgaaaag attgttaaaa ttcataaata 60
cttaagcact gtctacaatt atttacaatt tttggggcta catcattaca atgcaataat 120
gtcaaagaat cataataaaa ttatttctgt cgaaataatt agatgcaagg atctcttgga 180
taaaaacgtg catgcggaaa tccttaagaa agtttagact tggcagcttt acaatctcga 240
atcaacttaa atttatcgaa cctgatatgc gtatgcatat ataaacacac atgctagaca 300
aaaaaaagta tttgaatttt atagttgaca aaaaatatat tcgaattata tgtatagata 360
atgagtttgt agtgacaagc atgatgatgc attgagatca cgataaggga taattgaaca 420
gtaaatttgc gatgaaaaca ttaaaataac taaaaacagt tgacacacca ttttctccgc 480
gaagaagaaa attcataaaa tctaataaaa agtcacgatc atgtattaaa ataaaagatt 540
attatgtcgg ctctacacca aaataattta tctcgttata tcgatgtttt cttgattctt 600
gaattctaaa ggggactttt atttatttat aaattgagaa ctttaatgaa atttgctttt 660
aggaaacacg agagatgatt agttgcaaat aagatcctca tcttctacat aattacatta 720
gctctaacct tatttttcct ttgtttcaaa aatcataata acctcttaaa ataaataata 780
caattttagc taagtattat agcagaataa taatctctat gacacatgat aaacacttta 840
agtactatac acgattaaca accataaaac gctgaatata tggtttgata atttagtgat 900
gtgtattttt accttttat ctttttaaca aaataataac acatatatga ttaatgaata 960
atgtattgaa tttgtttctg ctatatttta ctattaataa catcataacg ttttcatgtt 1020
tgtttgacaa aaaagttttc atgctttcaa aatttgatga tgttatatgc tataactgta 1080
ttagaaatat aattagataa aaacgaaaca ttattaatat ataaaaacaa atcttatatt 1140
gtttagctcg acatttagat tattgttttc tgaagttatt tttgtaaact ttttatcttc 1200
aaattgaata actaatcata aaattagtta ttaattaatg tatggctgat aaaaaaagaa 1260
aacaagtctt atactagctt tttataataa cttgtggata ctctattata aagagttttt 1320
acttcttgat aattgagttg ttacatactc ctattattca tacgacatac aagtcgtatt 1380
tatagggtta ggataagtta ccaaaatgtg gaatcacttg caataaatgt gaaaacttgt 1440
ggactgttgt cacaactatt tattatattt agatctcaca caaaatttgt tctgtcaagt 1500
gaatgtatcc aacatgaaat tggcaaaagc cagctataaa caaaacccaa aaaaaaaaac 1560
cacctctctc aatagtaaac aaaaaatagg ataatagggt catctaaaca caagcaaata 1620
tggacttatg ctgttgtcat gatagataaa aatatgtacg ggtcttttaa gagcatcaaa 1680
tcaatttagc ccgtgggtcc tttttctttt tacttactta aagacttagt gcaatatttg 1740
tcccatatct ccctttaccc ctaccacacc ccacctccac cctcaaacaa ttgttgcata 1800
taaataagga agtgattgat aaatgtagag acaaataccc atcaaactct gtctctcact 1860
ttccttcatt atttactcat tgcccaaaat aattcattta tcatatcctc aaactttttt 1920
ttttctatac ctcgaaatcc aaaaaaataa atacattggt ttagtgtggc attgatgttt 1980

ttggtataaa gctcttcacc a 2001

<210> 5
<211> 2000
<212> DNA
<213> Arabidopsis thaliana

<400> 5
caatttgttt agaaccgttt tctatttctt attattattt gcttttgtcg ttgaaatgcg 60

ttttagtttt tcaatttgct tgttaatgtt ttaaattcac catcttgtct tcttccttag 120

aaaagtctcg cactccatct tttacagttg attttattcc gacaaaaata cattacacgt 180

actctttctc gtttcggatt atttgttttt gtatggattt tagttttcca tctctcgagg 240

tcagcttttt tttttccaca actcgtgaga cgtggtgact tgtgactttt ttgacaaatc 300

ttactctggc gaatgatttc tttatttgtc cgaaatattg tctagcagat gagaaaattc 360

agctgaaaaa aaaaacagta aaatagtgta catctacttc agcgttgaag cataattgtt 420

gaataaaacg aatgtatcct ttatcaaaat atcttttgtc tattacgttg tcatctaaat 480

tactgtatga tgttaagtta acgtgggata tttatcgatc ttttttggc aaatattatt 540

taatcttttg ttatttaaat gagagttctt taaatttgtc acattaaaat tcattaataa 600

taactaataa atagtgcata gaaaacttac aaaataattt ttttaagaca attttatttt 660

ctaaaaaatc aaataataag aaatagagga agtattactt tccaaaaaaa ctaacttgac 720

ctacattagt atataaactt caaaacaaat aattgaatat cattggttta aaattataga 780

aaaatataaa tcacaaaact aatacatttc tgatcagttt taatcacaaa aactaataca 840

tttttttaat ttgtgtgttt ttctataaaa tcaattaaaa atgaactata tggcaatatt 900

gaattaaaac aagtggcaag aatttaattg tgtaatttat gttagagatc aaaattttct 960

ttccagaaat tctgtgtaca atatatattc actttagaag ttttaactaa caaaaacaaa 1020

tttgttttta aacaaaagaa aaaaaaattg tgagaaattt aatatgaaat atctggttat 1080

acaattagag attaaacagc attatagata aataaattat atactagtat gagttttcag 1140

ccacacacaa aatagtttct gtctttctga accttgaaag gtggtacatt tcatattttc 1200

ctaagaatat ttttaagttc gtctccaaaa ttccagaact tccataatat atataaaata 1260

ctattgttaa tgttatatta ttttttccta tttttaattg ttccaacata ctattgaacg 1320

ttttaaacag tattatctta tatgtttcat gaattccaaa actaatgtgg attttagtct 1380

ataacgattc gatattagta aaaatgatga ttacattggc taaaagaatt aaaaatcact 1440

ggagtaatat attgtataat ttggtaatga taaatttaat tcataggaga aacaatatat 1500

ggctaggtaa gaaataaaga atacgtacaa gaacctccat ctcaatttat tttccactga 1560

caaagtctac acattttaat caaatgagat atttatttat aaccaatgaa aaactacaaa 1620

tccacgtgtc aaacacttga aaacgacggc gtcggctaca cacgagccat gccaaatcaa 1680

gataaaaagc tctacgttcc ttctttaaat ttcttacttg gctgaacata caaaactccc 1740

tcttctctat cttcttcatc ttaaagaaaa aataagagat attcgtaaag agagaacaca 1800

```
aaatttcagt ttacgaaaag ctagcaaagt cgagtatcga ggaataacag aataagacgt    1860
atctatcctt gccttaatgt tcttaccaaa agatctagtc ctttctttgt atgatcgatc    1920
catcacaagc ccacaacaac aacaactaca tctctttctc tatctctagc ttctattatt    1980
aatacattca agaatcaaga                                                2000
```

<210> 6
<211> 2236
<212> DNA
<213> Arabidopsis thaliana

<400> 6

```
atgggattga cctcatcctt acggttccat agacaaaaca acaagacttt cctcggaatc      60
ttcatgatct tagttctaag ctgtatacca ggtagaacca atctttgttc caatcattct     120
gttagtaccc caaaagaatt accttcttca aatccttcag atattcgttc ctcattagtt     180
tcactagatt tggagggtta tataagcttc gacgatgtcc acaatgtggc caaggacttt     240
ggcaacagat accagttacc acctttggca attctacatc caaggtcagt tttgatattt     300
tcatcgatga tgaagcatat agtacatctg ggctccacct caaatcttac agtagcagct     360
agaggccatg gtcactcgct tcaaggacaa gctctagctc atcaaggtgt tgtcatcaaa     420
atggagtcac ttcgaagtcc tgatatcagg atttataagg ggaagcaacc atatgttgat     480
gtctcaggtg gtgaaatatg gataaacatt ctacgcgaga ctctaaaata cggtctttca     540
ccaaagtcct ggacagacta ccttcatttg accgttggag gtacactatc taatgctgga     600
atcagcggtc aagcattcaa gcatggaccc caaatcaaca acgtctacca gctagagatt     660
gttacaggta tttcattcat gctttatctc tgcggtagtc tcaaaaaaat atgcacctgt     720
aaagaatatc catctcttca tgagcaaaaa cactgacgac tttaaataat ttttgactat     780
aaaacaagag tgcataggca caaatgtgaa atatgcaaca cacaattgta acttgcacca     840
agaaaaaagt tataaaaaca aacaactgat aagcaatata tttccaatat ttaatcaggg     900
aaaggagaag tcgtaacctg ttctgagaag cggaattctg aacttttctt cagtgttctt     960
ggcgggcttg gacagtttgg cataatcacc cgggcacgga tctctcttga accagcaccg    1020
catatggtaa agttctatct tgaacaaagt tcaaacaata tacgctatga ttctaagaac    1080
cactttcctg acacagtcaa ataactttta ataggttaaa tggatcaggg tactctactc    1140
tgacttttct gcattttcaa gggaccaaga atatctgatt tcgaaggaga aaacttttga    1200
ttacgttgaa ggatttgtga taatcaatag aacagacctt ctcaataatt ggcgatcgtc    1260
attcagtccc aacgattcca cacaggcaag cagattcaag tcagatggga aaactcttta    1320
ttgcctagaa gtggtcaaat atttcaaccc agaagaagct agctctatgg atcaggtaag    1380
atgtgaaagc aatatataac tagacttagt ttccacagag agctccaaat caaccgttgg    1440
ctactagcct actaacataa tgaatggttg ccgtgcagga aactggcaag ttactttcag    1500
agttaaatta tattccatcc actttgtttt catctgaagt gccatatatc gagtttctgg    1560
```

atcgcgtgca tatcgcagag agaaaactaa gagcaaaggg tttatgggag gttccacatc 1620
cctggctgaa tctcctgatt cctaagagca gcatatacca atttgctaca gaagttttca 1680
acaacattct cacaagcaac aacaacggtc ctatccttat ttatccagtc aatcaatcca 1740
agtaagtgag caaaatgcca aaagcaaatg cgtccagtga ttctgaaaca taaattacta 1800
accatatcca acattttgtg gtttcaggtg gaagaaacat acatctttga taactccaaa 1860
tgaagatata ttctatctcg tagccttttct cccctctgca gtgccaaatt cctcagggaa 1920
aaacgatcta gagtaccttt tgaaacaaaa ccaaagagtt atgaacttct gcgcagcagc 1980
aaacctcaac gtgaagcagt atttgcccca ttatgaaact caaaaagagt ggaaatcaca 2040
ctttggcaaa agatgggaaa catttgcaca gaggaaacaa gcctacgacc ctctagcgat 2100
tctagcacct ggccaaagaa tattccaaaa gacaacagga aaattatctc ccatccaact 2160
cgcaaagtca aaggcaacag gaagtcctca aaggtaccat tacgcatcaa tactgccgaa 2220
acctagaact gtataa 2236

<210> 7
<211> 2991
<212> DNA
<213> Arabidopsis thaliana

<400> 7
atggctaatc ttcgtttaat gatcacttta atcacggttt taatgatcac caaatcatca 60
aacggtatta aaattgattt acctaaatcc cttaacctca ccctctctac cgatccttcc 120
atcatctccg cagcctctca tgacttcgga aacataacca ccgtgacccc cggcggcgta 180
atctgcccct cctccaccgc tgatatctct cgtctcctcc aatacgccgc aaacggaaaa 240
agtacattcc aagtagcggc tcgtggccaa ggccactcct taaacggcca agcctcggtc 300
tccggcggag taatcgtcaa catgacgtgt atcactgacg tggtggtttc aaaagacaag 360
aagtacgctg acgtggcggc cgggacgtta tgggtggatg tgcttaagaa gacggcggag 420
aaaggggtgt cgccggtttc ttggacggat tatttgcata taaccgtcgg aggaacgttg 480
tcgaatggtg gaattggtgg tcaagtgttt cgaaacggtc ctcttgttag taacgtcctt 540
gaattggacg ttattactgg tacgcatctt ctaaactttg atgtacatac aacaacaaaa 600
actgtttttg ttttatagta tttttcattt tttgtaccat aggttttatg ttttatagtt 660
gtgctaaact tcttgcacca cacgtaagtc ttcgaaacac aaaatgcgta acgcatctat 720
atgtttttttg tacatattga atgttgttca tgagaaataa agtaattaca tatacacaca 780
tttattgtcg tacatatata aataattaaa gacaaatttt cacaattggt agcgtgttaa 840
tttgggattt ttgtaatgta catgcatgac gcatgcatat ggagcttttc ggttttctta 900
gatttgtgta gtatttcaaa tatatcattt attttctttc gaataaagag gtggtatatt 960
tttaaaatag caacatttca gaatttttct ttgaatttac actttttaaa ttgttattgt 1020
taatatggat tttgaataaa taatttcagg gaaaggtgaa atgttgacat gctcgcgaca 1080
gctaaaccca gaattgttct atggagtgtt aggaggtttg ggtcaatttg gaattataac 1140

gagagccaga attgttttgg accatgcacc taaacgggta cgtatcatca tattttacca 1200
tttgttttag tcagcattca tttttcatta gtaattccgt ttcaatttct aaattttttt 1260
agtcaataga aaatgattct tatgtcagag cttgattatt tagtgatttt tattgagata 1320
aaataaaata taacctaacg gaaataatta ttttactaat cggataatgt ctgattaaaa 1380
cattttatga tattacacta agagagttag agacgtatgg atcacaaaac atgaagcttt 1440
cttagatggt atcctaaaac taaagttagg tacaagtttg gaatttaggt caaatgctta 1500
agttgcatta atttgaacaa aatctatgca ttgaataaaa aaaagatatg gattatttta 1560
taaagtatag tccttgtaat cctaggactt gttgtctaat cttgtcttat gcgtgcaaat 1620
ctttttgatg tcaatatata atccttgttt attagagtca agctctttca ttagtcaact 1680
actcaaatat actccaaagt ttagaatata gtcttctgac taattagaat cttacaaccg 1740
ataaacgtta caatttggtt atcattttaa aaaacagatt tggtcataat atacgatgac 1800
gttctgtttt agtttcatct attcacaaat tttatataat tattttcaag aaaatattga 1860
aatactatac tgtaatatgg tttctttata tatgtgtgta taaattaaat gggattgttt 1920
tctctaaatg aaattgtgta ggccaaatgg tttcggatgc tctacagtga tttcacaact 1980
tttacaaagg accaagaacg tttgatatca atggcaaacg atattggagt cgactattta 2040
gaaggtcaaa tatttctatc aaacggtgtc gttgacacct ctttttttccc accttcagat 2100
caatctaaag tcgctgatct agtcaagcaa cacggtatca tctatgttct tgaagtagcc 2160
aagtattatg atgatcccaa tctccccatc atcagcaagg tactacacat ttacattttc 2220
atcatcgttt ttatcatacc ataagatatt taaatgattc atcattgcac cacattaaga 2280
tattcatcat catcatcgtt acattttttt ttgcatctta tgcttctcat aatctactat 2340
tgtgtaggtt attgacacat taacgaaaac attaagttac ttgcccgggt tcatatcaat 2400
gcacgacgtg gcctacttcg atttcttgaa ccgtgtacat gtcgaagaaa ataaactcag 2460
atctttggga ttatgggaac ttcctcatcc ttggcttaac ctctacgttc ctaaatctcg 2520
gattctcgat tttcataacg gtgttgtcaa agacattctt cttaagcaaa aatcagcttc 2580
gggactcgct cttctctatc caacaaaccg gaataagtac atacttctct tcattcatat 2640
ttatcttcaa gaaccaaagt aaataaattt ctatgaactg attatgctgt tattgttaga 2700
tgggacaatc gtatgtcggc gatgatacca gagatcgatg aagatgttat atatattatc 2760
ggactactac aatccgctac cccaaaggat cttccagaag tggagagcgt taacgagaag 2820
ataattaggt tttgcaagga ttcaggtatt aagattaagc aatatctaat gcattatact 2880
agtaaagaag attggattga gcattttgga tcaaaatggg atgatttttc gaagaggaaa 2940
gatctatttg atcccaagaa actgttatct ccagggcaag acatcttttg a 2991

<210> 8
<211> 3301
<212> DNA
<213> Arabidopsis thaliana

<400> 8
atggcgagtt ataatcttcg ttcacaagtt cgtcttatag caataacaat agtaatcatc 60
attactctct caactccgat cacaaccaac acatcaccac aaccatggaa tatcctttca 120
cacaacgaat tcgccggaaa actcacctcc tcctcctcct ccgtcgaatc agccgccaca 180
gatttcggcc acgtcaccaa aatcttccct tccgccgtct taatcccttc ctccgttgaa 240
gacatcacag atctcataaa actctctttt gactctcaac tgtcttttcc tttagccgct 300
cgtggtcacg gacacagcca ccgtggccaa gcctcggcta aagacggagt tgtggtcaac 360
atgcggtcca tggtaaaccg ggatcgaggt atcaaggtgt ctaggacctg tttatatgtt 420
gacgtggacg ctgcgtggct atggattgag gtgttgaata aaactttgga gttagggtta 480
acgccggttt cttggacgga ttatttgtat ttaacagtcg gtgggacgtt atcaaacggc 540
ggaattagtg gacaaacgtt tcggtacggt ccacagatca ctaatgttct agagatggat 600
gttattactg gtacgtacca cgatcttttt cacacagaga ttaaaaaaaa cagtaatagt 660
gattttaact tcgtacgttt ctgatagaca acaaagaact tcgtacgttt ttcgaagttt 720
tttcgtcttt ttcattttag atctgcgcgg ccatttttgg ttatgctatt gtttgtttgt 780
attgtttgtc tctgtttatt tatttctcga acttgttgat agcttttctt cttttcacac 840
atcaatctaa tcaccttttt tggtcttaag attagaaaga agatacggac taggtaaaaa 900
taggtggttg taaacgtaga cgcattaaaa aaatattggt ttttttattt tttgataagc 960
aaaattggtg gttggtctaa gattataaac ttgatattaa tgcaaaggtc gatctagcaa 1020
tagaagatta atcaatattc ttggtgtttt aacaacagat tatttcatca ttaaaatcgt 1080
gaaacaaaga aattttggta gtatacatta cgtgtagttt tgttagttta ttaaaaaaaa 1140
tagtatatag ttttgttaaa acgcgattta tttagtaaca cattagtata ttacacgttt 1200
aaccaactaa actttttttt ttgaataatt atgttctata tttcttactc aaattatgca 1260
aatttcgtgg attcgaagtc aaatttctgc gaaatttaca tggtcatata ttataaaact 1320
gttcatataa cccggtgaac aaacagacaa ttaagggttt gaatggttac ggcggttggg 1380
gcggacacaa ccgtcaatag atcagaccgt tttttattta ccattcatca attatattcc 1440
gcagtggttt ggggtaaaaa aaatagaaga aaaccgcagc ggaccaattc cataccgttt 1500
ttacatacaa ataaacatgg tgcgcaacgg tttattgtcc gcctcaaaaa tgaaatggac 1560
taaaccgcag ataaattaga ccgctttgtc cgctgcctcc attcatagac taaaaaaaaa 1620
caaccaaaaa aaaaatggtc ccacgcccat gattttacac gaggtttctt gtggcgtaag 1680
gacaaaactc aaaagttcat aacgtttggt cctaaccagg tgtaatggat taagtaacag 1740
tcaattttct tattatagct gtatccatta tgtccacata tgcatccata tacattacac 1800
tgttggtctc aagtgtagtt agattacgaa gactttcaag ttccattttt tggttaggag 1860
ataaacataa tttaatgata ccgactttag cactctaggc tcaaaacaag tacagaagag 1920
aatagtttta tttcaaactc gttgcattgt tgtatcaatt aattgtgtta gtctttgtat 1980

attcttacat aacggtccaa gtttgttgaa atagtttact tactaaactt ttcctaatgg 2040
ggtcaaattt tattttatag gaaaaggaga gattgcaact tgttccaagg acatgaactc 2100
ggatcttttc ttcgcggtgt taggaggttt gggtcaattc ggcattataa caagagccag 2160
aattaaactt gaagtagctc cgaaaagggt atgttaaatt tgtaaattat gcaactacag 2220
aaaattctat gaaatttatg aatgaacata tatgcatttt tggatttttg taggccaagt 2280
ggttaaggtt tctatacata gatttctccg aattcacaag agatcaagaa cgagtgatat 2340
cgaaaacgga cggtgtagat ttcttagaag gttccattat ggtggaccat ggcccaccgg 2400
ataactggag atccacgtat tatccaccgt ccgatcactt gaggatcgcc tcaatggtca 2460
aacgacatcg tgtcatctac tgccttgaag tcgtcaagta ttacgacgaa acttctcaat 2520
acacagtcaa cgaggtccgt acatacatac aatcataaat catacatgta taattgggag 2580
atctttatgc attattcaat tatattaatt tactttagtt atttaactta tgcaggaaat 2640
ggaggagtta agcgatagtt taaaccatgt aagagggttt atgtacgaga aagatgtgac 2700
gtatatggat ttcctaaacc gagttcgaac cggagagcta aacctgaaat ccaaaggcca 2760
atgggatgtt ccacatccat ggcttaatct cttcgtacca aaaactcaaa tctccaaatt 2820
tgatgatggt gtttttaagg gtattatcct aagaaataac atcactagcg gtcctgttct 2880
tgtttatcct atgaatcgca acaagtaagt ttaactcgat attgcaaaat ttactatcta 2940
cattttcgtt ttggaatccg aaatattctt acaagctaat tttatgcggc gtttttaggt 3000
ggaatgatcg gatgtctgcc gctatacccg aggaagatgt attttatgcg gtagggtttt 3060
taagatccgc gggttttgac aattgggagg cttttgatca agaaaacatg gaaatactga 3120
agttttgtga ggatgctaat atgggggtta tacaatatct tccttatcat tcatcacaag 3180
aaggatgggt tagacatttt ggtccgaggt ggaatatttt cgtagagaga aaatataaat 3240
atgatcccaa aatgatatta tcaccgggac aaaatatatt tcaaaaaata aactcgagtt 3300
a 3301

<210> 9
<211> 2782
<212> DNA
<213> Arabidopsis thaliana

<400> 9
atgactaata ctctctgttt aagcctcatc accctaataa cgctttttat aagtttaacc 60
ccaaccttaa tcaaatcaga tgagggcatt gatgttttct tacccatatc actcaacctt 120
acggtcctaa ccgatccctt ctccatctct gccgcttctc acgacttcgg taacataacc 180
gacgaaaatc ccggcgccgt cctctgccct tcctccacca cggaggtggc tcgtctcctc 240
cgtttcgcta acggaggatt ctcttacaat aaaggctcaa ccagccccgc gtctactttc 300
aaagtggctg ctcgaggcca aggccactcc ctccgtggcc aagcctctgc acccggaggt 360
gtcgtcgtga acatgacgtg tctcgccatg gcggctaaac cagcggcggt tgttatctcg 420
gcagacggga cttacgctga cgtggctgcc gggacgatgt gggtggatgt tctgaaggcg 480

```
gcggtggata gaggcgtctc gccggttaca tggacggatt atttgtatct cagcgtcggc     540
gggacgttgt cgaacgctgg aatcggtggt cagacgttta gacacggccc tcagattagt     600
aacgttcatg agcttgacgt tattaccggt acgtaaatac caaaacttca ctaatctcgt     660
tacaattttt taattttttg gtaatataaa ttttgtacgg ctcaactctt aattaagaat     720
gaaacagtat ctatgatctt ctagatgctc tttttttgtc tgcaagcttt aattgtagta     780
acatcagcga tatatatatc acatgcatgt gtattattga tgataatata taatgtttta     840
gttacaaatt tgattctcaa ggtaaaactc acacgccata accagtataa aactccaaaa     900
atcacgtttt ggtcagaaat acatatcctt cattaacagt agttatgcta taatttgtga     960
ttataaataa ctccggagtt tgttcacaat actaaatttc aggaaaaggt gaaatgatga    1020
cttgctctcc aaagttaaac cctgaattgt tctatggagt tttaggaggt ttgggtcaat    1080
tcggtattat aacgagggcc aggattgcgt tggatcatgc acccacaagg gtatgtatca    1140
tgcatctata gtgtaatcaa tttataattt taatgtagtg gtcctaaatc caaaatttga    1200
tttgatttgg ttggaacgta cgtatatata ataagtcaaa aggctgattt tgaagacgaa    1260
tttatatact tttgttgaat taaatctgat tttgcttacg ttttattaga ttctgcgtaa    1320
taaatcctag gacttgctcg agtgtaatct tgtcttatgc ttgcaaatct tgttgatgtc    1380
aatatctaat ctttttttatt atatttccct acgtaagttt tagatatagt tattttaaac    1440
tgctataaat tgtgtacgta tagactttag ataaaaagtt gtggtcgctt gcacctattt    1500
gtttatcgct atagtgattc aaaggtctat atatgattct tggtttttct ttttgaaaaa    1560
aatagaccat acaatccaag gaagatgatc ttaaatggac taatttatgg atataaattg    1620
atatacaaat ctgcaggtga aatggtctcg catactctac agtgacttct cggcttttaa    1680
aagagaccaa gagcgtttaa tatcaatgac caatgatctc ggagttgact ttttggaagg    1740
tcaacttatg atgtcaaatg gcttcgtaga cacctctttc ttcccactct ccgatcaaac    1800
aagagtcgca tctcttgtga atgaccaccg gatcatctat gttctcgaag tagccaagta    1860
ttatgacaga accacccttc ccattattga ccaggtacta aaatccatta ttcatgatga    1920
ttatcttcac acaatcagta tcatcaccaa ttaccatcat cacttgtcat atatgatcca    1980
aagtaaatat atcacatgat ataaataaat cgttcaaatc ttttttttta aagaataaaa    2040
gaatcatttt caagcattac tcatacacat ctacgaatca ccgtgaccat atataaccat    2100
acgcttatta aataatcatt tttgtttgta ggtgattgac acgttaagta gaactctagg    2160
tttcgctcca gggtttatgt tcgtacaaga tgttccgtat ttcgatttct tgaaccgtgt    2220
ccgaaacgaa gaagataaac tcagatcttt aggactatgg gaagttcctc atccatggct    2280
taacatcttt gtcccggggt ctcgaatcca agattttcat gatggtgtta ttaatggcct    2340
tcttctaaac caaacctcaa cttctggtgt tactctcttc tatcccacaa accgaaacaa    2400
gtaaatattt actttttgat tttgttttat ttgaaagtat atcccaataa tgtatgttaa    2460
attgttaaca agaatttatt ttattaatag atggaacaac cgcatgtcaa cgatgacacc    2520
```

ggacgaagat gtttttatg tgatcggatt actgcaatca gctggtggat ctcaaaattg 2580
gcaagaactt gaaaatctca acgacaaggt tattcagttt tgtgaaaact cgggaattaa 2640
gattaaggaa tatttgatgc actatacaag aaaagaagat tgggttaaac attttggacc 2700
aaaatgggat gatttttaa gaaagaaaat tatgtttgat cccaaaagac tattgtctcc 2760
aggacaagac atatttaatt aa 2782

<210> 10
<211> 2805
<212> DNA
<213> Arabidopsis thaliana

<400> 10
atgacgtcaa gctttcttct cctgacgttc gccatatgta aactgatcat agccgtgggt 60
ctaaacgtgg gccccagtga gctcctccgc atcggagcca tagatgtcga cggccacttc 120
accgtccacc cttccgactt agcctccgtc tcctcagact tcggtatgct gaagtcacct 180
gaagagccat tggccgtgct tcatccatca tcggccgaag acgtggcacg actcgtcaga 240
acagcttacg gttcagccac ggcgtttccg gtctcagccc gaggccacgg ccattccata 300
aacggacaag ccgcggcggg gaggaacggt gtggtggttg aaatgaacca cggcgtaacc 360
gggacgccca agccactcgt ccgaccggat gaaatgtatg tggatgtatg gggtggagag 420
ttatgggtcg atgtgttgaa gaaaacgttg gagcatggct tagcaccaaa atcatggacg 480
gattacttgt atctaaccgt tggaggtaca ctctccaatg caggaatcag tggtcaagct 540
tttcaccatg gtcctcaaat tagtaacgtc cttgagctcg acgttgtaac tggttagtat 600
taaaacattc aagttcatat attttaaatg cttttgtctg aagttttact aataacaaga 660
aattgatacc aaaaagtagg gaaaggagag gtgatgagat gctcagaaga agagaacaca 720
aggctattcc atggagttct tggtggatta ggtcaatttg ggatcatcac tcgagcacga 780
atctctctcg aaccagctcc ccaaagggta atattttttt aatgactagc tatcaaaaat 840
ccctggcggg tccatacgtt gtaatctttt tagtttttac tgttgatggt atttttata 900
tattttggat aataaaaccc taaaatggta tattgtgatg acaggtgaga tggatacggg 960
tattgtattc gagcttcaaa gtgtttacgg aggaccaaga gtacttaatc tcaatgcatg 1020
gtcaattaaa gtttgattac gtggaaggtt ttgtgattgt ggacgaagga ctcgtcaaca 1080
attggagatc ttctttcttc tctccacgta accccgtcaa gatctcctct gttagttcca 1140
acggctctgt tttgtattgc cttgagatca ccaagaacta ccacgactcc gactccgaaa 1200
tcgttgatca ggtcactttc attattcact tagaaaaaag cgatattttc atttttata 1260
ttgatgaata tctggaagga tttaacgcta tgcgactatt gggaaatcat tatgaaaaaa 1320
tatttagttt atatgattga aagtggtctc catagtattt ttgttgtgtc gactttatta 1380
taacttaaat ttggaagagg acatgaagaa gaagccagag aggatctaca gagatctagc 1440
ttttccacct gaacttaata atgcacattt atataattat ttttcttctt ctaaagttta 1500

gtttatcact agcgaattaa tcatggttac taattaagta gtggacaggg tcatggacca 1560
ctcactcacc aaataatgat tcctctttac tcttaagttt aattttaata aaaccaactc 1620
tactggaatc ttaacttatc cttggttttg gtaggctttt atagcaacac ggttttttta 1680
attttcctat tccagatttt gtatattaaa tgtcgatttt ttttcttttt gtttcaggaa 1740
gttgagattc tgatgaagaa attgaatttc ataccgacat cggtctttac aacggattta 1800
caatatgtgg actttctcga ccgggtacac aaggccgaat tgaagctccg gtccaagaat 1860
ttatgggagg ttccacaccc atggctcaac ctcttcgtgc caaaatcaag aatctctgac 1920
ttcgataaag gcgttttcaa gggcattttg ggaaataaaa caagtggccc tattcttatc 1980
taccccatga acaaagacaa gtaagtcttg acattaccat tgattactac ttctaaattt 2040
cttctctaga aaaaagaata aaacgagttt tgcattgcat gcatgcaaag ttacacttgt 2100
ggggattaat tagtggtcca agaaaaaaag tttgtcaaaa ttgaaaaaaa ctagacacgt 2160
ggtacatggg attgtccgaa aaacgttgtc cacatgtgca tcgaaccagc taagattgac 2220
aacaacactt cgtcggctcg tatttctctt tttgttttgt gaccaaatcc gatggtccag 2280
attgggttta tttgttttta agttcctaga actcatggtg ggtgggtccc aatcagattc 2340
tcctagacca aaccgatctc aacgaaccct ccgcacatca ttgattatta cattaatata 2400
gatattgtcg ttgctgacgt gtcgtaattt gatgttattg tcagatggga cgagaggagc 2460
tcagccgtga cgccggatga ggaagttttc tatctggtgg ctctattgag atcagcttta 2520
acggacggtg aagagacaca gaagctagag tatctgaaag atcagaaccg tcggatcttg 2580
gagttctgtg aacaagccaa gatcaatgtg aagcagtatc ttcctcacca cgcaacacag 2640
gaagagtggg tggctcattt tggggacaag tgggatcggt tcagaagctt aaaggctgag 2700
tttgatccgc gacacatact cgctactggt cagagaatct ttcaaaaccc atctttgtct 2760
ttgtttcctc cgtcgtcgtc ttcttcgtca gcggcttcat ggtga 2805

<210> 11
<211> 1975
<212> DNA
<213> Arabidopsis thaliana

<400> 11
atgagctatc tacatgcaag cctcctcagg aaaagaacca tgcttatagt aagaagtttc 60
accatcttgc ttctcagctg catagccttt aagttggctt gctgcttctc tagcagcatt 120
tcttctttga aggcgcttcc cctagtaggc catttggagt ttgaacatgt ccatcacgcc 180
tccaaagatt ttggaaatcg ataccagttg atccctttgg cggtcttaca tcccaaatcg 240
gtaagcgaca tcgcctcaac gatacgacac atctggatga tgggcactca ttcacagctt 300
acagtggcag cgagaggtcg tggacattca ctccaaggcc aagctcaaac aagacatgga 360
attgttatac acatggaatc actccatccc cagaagctgc aggtctacag tgtggattcc 420
cctgctccat atgttgatgt gtctggtggt gagctgtgga taaacatttt gcatgagacc 480
ctcaagtacg ggcttgcacc aaaatcatgg acggattacc tgcatttaac tgtaggtggt 540

actctgtcca atgctggaat aagcggccag gcattccgac atggaccaca gatcagcaat 600
gttcatcaac tggagattgt cacaggttag ttcagagttg cagtattcgt gttttgaaag 660
catagactct atatggttgg tgactattaa caacatgaag agattcccga gaatagctac 720
ccactaatgt catgcctatt tattgactgc aggaaaaggc gagatcctaa actgtacaaa 780
gaggcagaac agcgacttat ttaatggtgt tcttggtggt ttaggtcagt ttggcatcat 840
aacgcgggca agaatagcat tggaaccagc accaaccatg gtaaacaata aataaataaa 900
aaacttaaaa actgaacacg cgtgtgtcct cctaactctg tataatggac aggtaaaatg 960
gataagagtg ttatacctgg attttgcagc ttttgccaag gaccaagagc aactaatatc 1020
tgcccagggc cacaaattcg attacataga agggtttgtg ataataaaca ggacaggcct 1080
cctgaacagc tggaggttgt ctttcaccgc agaagagcct ttagaagcaa gccaattcaa 1140
gtttgatgga aggactctgt attgtctgga gctagccaag tatttgaagc aagataacaa 1200
agacgtaatc aaccaggtga gaaaacagag tagaagcaat cggtagaatc ttctttggta 1260
gatgacattc attggaactg aaaatatata tatatttgtc caatccagga agtgaaagaa 1320
acattatcag agctaagcta cgtgacgtcg acactgttta caacggaggt agcatatgaa 1380
gcattcttgg acagggtaca tgtgtctgag gtaaaactcc gatcgaaagg gcagtgggag 1440
gtgccacatc catggctgaa cctcctggta ccaagaagca aaatcaatga atttgcaaga 1500
ggtgtatttg gaaacatact aacggataca agcaacggcc cagtcatcgt ctacccagtg 1560
aacaaatcaa agtaagaaag aaagaaagaa agagctagtc atgattttgt ttcttttcac 1620
ttgttgacaa aacaaaagca tgttggtgag caggtgggac aatcaaacat cagcagtaac 1680
accggaggaa gaggtattct acctggtggc gatcctaaca tcggcatctc cagggtcggc 1740
aggaaaggat ggagtagaag agatcttgag gcggaacaga agaatactgg aattcagtga 1800
agaagcaggg atagggttga agcagtatct gccacattac acgacaagag aagagtggag 1860
atcccatttc ggggacaagt ggggagaatt tgtgaggagg aaatccagat atgatccatt 1920
ggcaattctt gcgcctggcc accgaatttt tcaaaaggca gtctcatact catga 1975

<210> 12
<211> 1307
<212> DNA
<213> Arabidopsis thaliana

<400> 12
tttaaattta tccaaatatg gaagtgagcc cattccaaat aattttgggc ttatttgagt 60
tttagattaa taaggctagt attctatttt tgggtttgtt tagtatgtag tttttataag 120
gcccatcatg taattgcata ggttatgctg tattttgatt ttgcatttac catgtcaatg 180
ccaactcaca cactattgat ttgtttaata tattttccca ttgtgtattc tagaaattat 240
attttaatct aatttgttca tatgatgtat gagaatctta aagtagcata ttcttttgac 300
aaataacaaa gtagtatatt ttaaatgaat ttctcttgtt tacttgccat tatagtcatt 360

tatttaaatt atgttcaaaa tcagattacc ttacctacta attcacccta acgaaatttt 420
catgtcttta atttgaaaca atcgaagatt tgacaactta ttttacaatt tttctataat 480
aataatcttt tcgacgagtt aaaaaagaaa agttatattt tattttcctg ctcaaaatta 540
ttgtattctt gtccggaaaa cttaactgtc aaccaaatat ttataaattg tattctgtaa 600
aaaaaattct aaagggtaag aattcccctt gtttacgttt cctttgttta cgtcccacta 660
tattcattta tttaatttgt ttttggtcga cagcttatgt tactggatag gttcaaatga 720
attaaaccga ataaacgttg tttctatatg aaatagaatg tgcgtgaaca cgttccatca 780
gctttagaat tagttagtat agttaaatat acatgatctt acttcaatca attaatcaac 840
cacacatgat acaactatta attcttaaag tggtttccct tttgagaatt gagggcaaca 900
ctaccaatcc attaagccaa aaatgcaagg ggagattata cttatttgaa gaacgtggcc 960
aagatacaga tctttattac gtatagtgag aatctttctt ctcataaaag aaataagaaa 1020
gagaagaaaa aaacgagttg ccatatcatg taaactaaaa gtcttcccta tcgaccatca 1080
tccatatctt ccccaggtca ataaatacca tgaaatttcc ctaacataga tcactgcacc 1140
aattaaccat tacttacttc tcaggtaatc tctctacctt ttctcatatg attatagtta 1200
gtatagtatt tggttacatt atatatgata atctttttga aactatttgg ttacgtttct 1260
aatcgcaggt aataaatcgt aagatttatc aaacgcaagc aaaagag 1307

<210> 13
<211> 2533
<212> DNA
<213> Arabidopsis thaliana

<400> 13
tgatttacaa tatttttata ttggatcagc cgcgtaagga ggagatctga tctgatctga 60
tccacacacg tctcacgagt gtagtaggct cgacgtgctc aaactcaaag tgtgataacg 120
tattttttaa gcttaaaaga agtcatcatt attgttatct gtctcaatag aaataaacgc 180
cacgagatcc acgagtcgca ttttttttat tttgccaaaa gtcaaaaata tgtttccata 240
tatatcttcg gaaattaaga agaattagat tttaatctgc ggtataccgc ggggacaatt 300
tatcttttta aagttagtat aatttttttt tgcaaattat atctatttat aatatatttt 360
tattttatag tttacaatta ttattaagta acgtactgcc aaacccgtcc cgccaaaccc 420
atcccgtaaa aaaaccattt atttatatta ctgttgatct tatttatgat atatttataa 480
atcattgtct atatattttt gtcgatgcgg gacgttgaac ccacacattt tttgccaatt 540
ggttaatata tgttcatttt taaaattttg aatcataaaa tatgatggaa aaaattaaga 600
tttttttaaa ctctatatat tatataatga tgttaaaaat tatgatatat caaacttttt 660
ataagtttaa atattaataa tgttttataa attttaaata tataaataat aatatttaaa 720
aaattaatat gtttgtttat aaagtaatga cataataact ctaaaagcaa ggatttagaa 780
gatttaaatc tttaattaaa tattttcatt aataaataat taatgtcagt gacatgacat 840
tgtaaataag ttcaaataca aaatttattt atttaaacaa aaaacgttta gtaagaataa 900

tatttattta acaaaaaact ttgttttaaa atattgttaa caaatatgtt tatgctaatt 960
ttaagggatt gatttgaagt ttgttaggat taaatttgat tgataatgtg attgacaatt 1020
taaataaaaa gtttaaagta aaattaatta ttttggaaaa aaaaattaat gctaatgaca 1080
ttagatcgta aataagttca ggatttattt gctaaaatag ctacaaaaat gtatatatag 1140
atttcaaaat atactttttt aatcactatt ttttcgtgga gtagctgtct acgaatgttc 1200
tatccaatac attcgaacac gtgattgttc gttaattttc ttgattctgt aagagaaaca 1260
aaaaatatag atgtccaact ttttttttcg ggtgggaata tagacgtcca gcttagctac 1320
gtactgaata attcaaagtt ccaaactagt atatattaat acaattgacg ataaggtcat 1380
aaggatcgat ggattccaac gattcgatac aagtattaat gaaataagat aacacgattg 1440
tgacagcaaa ctctatattg atatttctat tttttaatta gccatgcgtt gcacgatcaa 1500
tttacaaaat aataaaagaa aatgatcgat caaagagcat tccattgaaa tttaattcca 1560
tcctgtaatc acataatttt gggcccaatc ctatttttca aatgtaacat gctattacat 1620
agtcacatag aacatcctaa aatagggtta aaatgtactt ttatctattt gcaattttga 1680
tattttcctt tctgaaaaag attagtatat ggcaaattat cttttagata aaagatcttt 1740
tgttctgact atacattaat ttattttaaa aaaaaaactt aacagatata tttgcaaata 1800
caaaatgatg aaaaataaaa gggataccat aatctaaaat ctgacaaaga aaatatacaa 1860
aaagtcaatt acgatactta gaaaaagaac tatatatttt tgggtaggga agttcaaaaa 1920
caaattaccg atttgctgac tatatgagca attattacat acttttattt atttgtacaa 1980
caattattac acatacttgt gtggaccaac atgattaatt ttatattggc catatggtgc 2040
gtagtaaatg ttataataac ttgaaattaa ataataacta agctcgactc gatatataga 2100
tccaaccagt agcctctctt attcacacct aatcttcatc ttcatcttcg cattcatagt 2160
ctctacgatc aggtaatccc cctctctcta tctatctttc atatatgtgt gtatgtgtaa 2220
actatctata ttctgaaaat agatcaatca attgatcttt tcctatctca attgttttca 2280
caaccatcag tttgactttt gatcgtttaa ggctcgagag aattatcatt cactgtagta 2340
aagatagttt ataccaacaa acccatttgg tgttgaccag ctttcaacat aagtatgagt 2400
tagagctaga accggattag tattaatgtt acttgtacct gttcatagta ctaaccaaaa 2460
atgatccaaa aaaatgaaaa taacaaataa accatttatg gttatcacag atagataaaa 2520
gaagtcaaca acg 2533

<210> 14
<211> 76
<212> DNA
<213> Arabidopsis thaliana

<400> 14
gattactcaa ttttctacta taaatagatt accaaatcat tcgatcggag acaatcacca 60
aagaaagaag tacaaa 76

<210> 15
<211> 842
<212> DNA
<213> Arabidopsis thaliana

<400> 15
tcagttgcac attaggagaa aagcttatat tcatactcag tatctcggca tctagtgtat 60

gatggaaaca attatcttta agactttagc attatttaaa gtgagaaaca agaaaacttc 120

gccataattg gagaattggt cattttgatc aaaatccaac accagactac gcgcagtccg 180

tttcgaagga ggtacactcc acttagtatc tagatttaat ttattataca gtatgtttaa 240

tttttttttt ttttcaaaca catatttttc ttcttaagaa tatatatgga aatataatat 300

gttaattatg taatgatcaa ataatattaa aacacacata tatatatata tataaaccat 360

atgagcaatt ggataacggg taaaatctaa ccaggtttca aaaattgtaa ctattgttga 420

tttttttttt tttttgatca acaactattg ttgagaaaaa catgtattat gtgagcaaag 480

gaaaaaaagg gaaaaaaaac tacagatcag taatatcaac tactccaatt caacttattt 540

gcttcatatt cacaacattc taatatttta atgactcgtg gatcacgaga ttatattaac 600

atttctatag attaattagt ggcctacatt caatgatatc aataattaca cattttatcc 660

agaaataaaa ataatcaata aatacgcatg aaggtcattg taatgcatat caataactcg 720

ttccctctct cctcaggtaa attctcagtc tctcgtctga taatagtttt gtagagtttg 780

gttaataaaa ctaatcgtgt acgctactaa tacgtacagg taactaatta atattcaaaa 840

ag 842

<210> 16
<211> 61
<212> DNA
<213> Arabidopsis thaliana

<400> 16
acacaacaaa cacaacttct acaagactca aatagtttct atttaattac taaaaagaaa 60

a 61

<210> 17
<211> 876
<212> DNA
<213> Arabidopsis thaliana

<400> 17
aaactaaaat ctcaacgtat gaattgtaca atttaaaatg caaatgttaa cttttttgga 60

tcaaatatat agttgttcgt attttctcag taaaaaaatc ctgcacaaag tttttatcta 120

gaatgtgtga ccatattgaa gtttttggcc ttctccaaac ataaaaagtt attagttaaa 180

agtatatgag ttggccaaaa aatacgaacc ggttcttgtt tctagattga gcgttacaga 240

aaattgtatt atatatatac tcttggttct aatttctctt aggtgatttc aattatactt 300

ttactgtttg aaataatgta caatgctgtg gaaacatgtt cttacaagac ttatcaaatc 360

gtcaaccaat tagagtccta aaaagggggt cactaattgt agaattctga cttatataaa 420

ttgcaacttg cacccttttac aagtaataac gtatataata aaattgacga ctctgtgaat 480

tttaaaagaa ctaattagct tgctgccatc gtttcattgg ccaacaccaa tataaagacg 540

attagttgag aagctgtgga agtaaaaaag gagtatatga atatttgaac atgtatttct 600

tccggtaatg ttttaaatat gcctcgtgga tcacgaaata atagattatt atttctgcta 660

gtacgatcaa tgatcctata tgcggatata tggttatatg caagaagtca atataaacac 720

ctatgattca cttaaatcga tcacaatcta ctcacttttg aaggtcatct ctcttttcct 780

ctctattagt acaaaattgg ttaataattg aaaattcaca atttgttttt ttttccaatt 840

atagtaacta tagtaagatt gatcagcaag caaaat 876

<210> 18
<211> 2265
<212> DNA
<213> Arabidopsis thaliana

<400> 18
tatacgatca aaattatagt aaatagtaac acttaatgag actttaaagt tgagatgtgt 60

aactagtcac cactcactca tgttagtgat tcatgtgtgt gtcagtgtgt gtgtgtgtgt 120

ttttatcaag gtcttctctt aagattcata agtgtgaatc tggtgggact taattggtct 180

ggtggagtag aacctatgcg ggcaatgagg aaggttaagg taggctaacc gagcccactc 240

tctaagtgtt taatttgtaa ccttaattcg taaataaaag ctgtttaatt tgtaaccttt 300

attagataaa attttgtctt tttttttgtc aaaagataaa atgttgtcaa tcattttaaa 360

aacaaagaaa aaaacttttt tggcaagaat ttatatattt gcttgtcata aattttccat 420

tttgtagtat tttgaaaaaa atattttaac gaatggaagc aataactcga caaaaagagt 480

caaactaaag taccttcaca tgcaaaacaa aacgtaaatt tggtattttt ttcccattaa 540

aaaaaatata tatatattct tggccttgtg tgcgaagacg atcgagaacg agaagaacga 600

taagctcaat acgcacgaac gtagatcgaa gactggaaag gtatatcttc atcaaaatat 660

tcatgattat ggttgagttt aattttgttt acgtatttgt tttaattttt caaaagtcca 720

ctgttggctg gctgttgcta tcttggaatg attttcatat ctcaataact attgtttact 780

ctaagttaaa ttattggggt gctatttatg atgactgatg aagtgatggg aacatgattg 840

caatattaaa atctcttttt tatttatatt aattataagt ttgtacccac ttcattttaa 900

atagataatg ccaagacgta ggtatattat tactgaattt attttttaat aataagtaaa 960

tatgcaggtt tcaactagaa aggtaacaca tcaacaagaa ataccaaaaa ccactagctt 1020

acatattttg atcatataag tattgaataa tatagttata gatgatcaaa tagttattat 1080

taattactta gctagtgtaa tttctatcga ttgtttcaca ttcgcaacag ataattatat 1140

aaataaatac atactacatg ttaacctttg tttttgttaa ataggatgtt ttcttgattg 1200

atgatctaga tcaactttta ataatttata cgtaccagct ttcattcgat cgtgcgactt 1260

cttggaatat cccatggtac atttgcagtc gagctagaaa gctttttttga taatttatga 1320

atcatattaa gcgtctctca attagattaa tatttagtac cagtgtaaat gatacaatta 1380
taaatctttt taccaacgaa tacagaattt gttttagaaa tattcttcgc aaaattttat 1440
aatatttcat aaatcagtta taaaaaaaaa aaaagcagtt ttgaattcag aggaattcgt 1500
aaatttccct aagagttatt aaaactagtt gtgatatttt ggagtattct ttatggctag 1560
atccatatga tccggttctt gtatctagat tttttttacg gaatattgta tgatatatct 1620
agtctcctct ttggttctaa acttctaatt aatcttcggt gatttgaatt aaacgttact 1680
gtttttaaat tatgattaag gctttaaaac atgttcatac aataattacc aaatcttaac 1740
caattagact cttaataagg agaacgtata tatatgttgc aacttgcaac tttagccctt 1800
tacaaataat taatcgtaca taatctaatt gataaacgat tctttaaatt ttaaaacaat 1860
gaattagctt gctgccatcg tttaatgggc caacgccaat ataaagacga ttagttgaga 1920
agttgtgcaa gtagaagtat atgaatattt tcattatcta aaaaaaaaaa tgtatatgaa 1980
catgcgtttt tcccagtaat gctttttata taaatatgcc tcgtggatca cgaaagaata 2040
aattattatt tcccttatta tttcatctag tacgatcaat atcaagaagt caatataaat 2100
aaacatacaa ttcactttaa ttgatcatag caccgataac aatctactct ctttaaaagg 2160
tcttatctct attcttctct attatgtaaa aattggttaa tagtagatat taacaattta 2220
tttttgcaac tacagaaatt cgtaagtact atcaagaagc agaag 2265

<210> 19
<211> 1458
<212> DNA
<213> Arabidopsis thaliana

<400> 19
aatataatgt ccaaatcatt tcacattagt catccggttt ataaaatatc ggaaaagaca 60
ttgaattgtt tatcccaact tttgtttatg tttcgagtgt atttttttg tcacaaaaat 120
gtttatttaa gccaaaacag agtcagtgta agagtggaag aagccacaca aacaaaaggg 180
taacttaaga actaaagaag acaagattat caaaaaggag cgtgacaatc actaaaacag 240
aatccagaaa aaactttatc tgttttagaa tccttattca actaatcaaa cttaagtaaa 300
cctgtaatag gaacaattaa ttatctcttt ttaggaagct tcagattcag aattcctcct 360
ctctcttcag cttgtagctt gtgcatttgg ttcctattat tcaagtgtga actaggacgt 420
ctagacgatt caccaacctc aaaatatctt ggcattcttg aagtgatatc ttcttcattt 480
aagggaaata ttagaccatg tggaagaaag gctgtcttct ccatgaagca atatagtatt 540
attcaatcga gactgacaaa ttctgaaata gtgataatgg aagcaatcga gaaagacaga 600
agaatagtca agaaaggcac tcccacgccc acatggcatt tcttttatcc tcctctcttc 660
atatttttgt agagtattaa tgatcaatta ataagagcaa ctccgatggt ttagaatcaa 720
ttggttttta attttcattt ttaaccttat tttatattaa aatgttgttt ttacaaagtt 780
aaaaaaaatt agaaaaaact taatttattt ctactccaat gattaagaac tattgggttc 840
ttaaaaaata acttggatac ccactcaata cacaacacat gtcctcttct taatattaag 900

aaaacatctt aattaagata taaatcttag ctaagatttg ttgtttgtat tatatttaat 960
cagtttaata gataagaatt agttaagaat tatctaagaa ccaaggttgg agttgctcta 1020
agttacagtg ttacacacta ttattcttaa tcaatccaaa atctaaattt ccataaacat 1080
gtattttatt tcaaatacct acaaatattg aactaaaact aaaactaaat ttatttcatt 1140
taacaaagac gaatgcaaaa aaacttagtg cacagtaatc atagtaatta aacaaagaca 1200
gatgccaaca aaagaaaaac taaaacacag taaacaaaga ccggtgccaa aaaaagaaga 1260
aaatgctatc ttttaaagag gaaacaacta gatttgattt gtttcacttt cacataaaaa 1320
atgaaagtca aaaacgtcct acaccattat ccaattattc aaattagccg agacacaaaa 1380
tcttctctat aaataataac actcacacac acaccaatcc tcttcataaa actcatcatt 1440
cctcattcat ctctaaca 1458

<210> 20
<211> 2042
<212> DNA
<213> Arabidopsis thaliana

<400> 20
caattattaa atttaaaaaa caatacaatt ttttttatg ttgtaaaata ctagaattga 60
acactataaa aaattaataa aaattgaaga aaacaattag tacagccata ttttacatat 120
gatatttata atatatatta gtaaatttat aaaattatta atttatacta ttgatgggag 180
catatattta tataagattt tcaataaaaa tattatctta ttagtttatc gatttatatc 240
aatttttata ttggtctcaa ctctgcacca aaaaaaatta ttaatttata aaaattatta 300
atttatcgaa tattaattta taaagatttt actgtatagt attattataa aagcgaaaga 360
aacatatatc atcccaaaaa ttctggttgt cgttacccgg tccttcttct tggcatcctc 420
taggtacttc ctctcgtgac ctccataaaa aaccatgagg atagatgtta agttttaaat 480
tttctctata ttgccaatct aacatcaagg gacctaactt ttatatatgt agttataaaa 540
ccccattact aactcaaatc acaaaataac ccaaagcgat gaaattggat aaacatgggc 600
ttttgtaatg atataggccg taattttttg tatattaaat cataatgggt ctttttcatg 660
agcctcttct gtagatgggc ttcatttatc agatatttat tttaacgcct gtaactatct 720
cataattgca aaattcgacc acgatttcga gaacaacccc ggccacttgt ttcttccatc 780
tgagacgctt atcttgaaaa cgtttagaaa ttaaccaaac ggatgttgat tgtttgactg 840
ttaaataaga gacaaaaaac aaaatataat agtctatgtg actacgatat aagctaataa 900
cgtgtttatg tttttcacct ttatatccat ttattaatgc gaccccaaaa tcagtgctcc 960
gtgtaaatgt atttgtcagt ttattcacat gatattaccc cttctacacc aatcaataaa 1020
atatactacc gctttatctt taagtatctt aaataatgtt aattctgttc atttcatatg 1080
aataataatg accaaaaaat atagttatcc taatggtttc tactagtatt tcaacattat 1140
agtctaaata ataagggatg aaacatataa acaaaaattc ggataaacaa atccatgtct 1200

attccgatat atttttataa tctggattag taattcgaaa aatgatgata aaagaatata 1260
atagtatgta tttttttaag ttttttatat atgctgtcat gaccccttat cttattcaag 1320
gagagtggta tgggtaacac aggattcaaa aaccgtggat ttttcgatat tgataaagag 1380
atgatatcaa tacattaaag attttcttta gtattaaaaa agattaaaga tgatcgttaa 1440
aagaaggaat caacggacgt gtcggggtaa aaaatatatt caatcgagac tgacaaattc 1500
ttaattaaat gaaagcaatc cagaaaacaa acaaaaaaga agaaaagtca agaaaggcac 1560
ccccacgccc acctgggtat tctatgaccc actctttaat gacctcttta tttattttaa 1620
ttaatcacgt acgaacgatt cattcatcaa ttgagttact taacagtaac acactatcat 1680
tcttaatcaa cccttacata taaaagttac ataagttgat taagattacc tgatatattt 1740
catacggaaa tctaaattta taaacacaaa cgaacatgtt atttatatag taagacaata 1800
actttatctc taacccaaaa gaatgaaaga aactagatct gctttgtgtc attttcacat 1860
aaatgagaag caaaatcacg aaaccataag agtgtcgtat gaattttgaa agtcaaaata 1920
acgtcacaca ccattatcca attattcaaa attagccgac aaaaaaacta aacaaatctc 1980
aaaaccttgt ctataaatac actcaatcct cttcttcaaa ctcaacatcg tcatctctaa 2040
ca 2042

<210> 21
<211> 1347
<212> DNA
<213> Arabidopsis thaliana

<400> 21
aattatagcc aatggttatt tgtggatgaa gaagatatgg gttgatgtaa tacttattta 60
taaacacgta agtatgatta ttccaatgga tattggagag taggtgaggc ggcttgatag 120
ctagtgtatg cttagcttgt gaactttaaa ctctcaatat ttatatgaat ttaagcatac 180
tacacattga gaagatgatc atgatcatat ctacgaacat tctacacacg tttctttgat 240
ttatatattt tggttaagct cagttttttt caaagtgttt gactaatctg aaggtggaag 300
ctattaactt aagagtcgag atcactgtgt cctgacgtgt ggattttgga ttaaaatttg 360
ctgctattct agtttgattt agcatgtttt caattttctc cgttcctgac aaattacaac 420
tcgaatttct taaaacaatt atgtttcgct caatggtttc aataacgttt tcagatatag 480
aaaatttgca aagaatgcaa accaagattt gtttatgctt tttgagtgtc tattgcagtt 540
caatttgact taaccaacct aattaatgga cacgtaatag atcatagact ttgatccgat 600
cctaatcctt acatgtcatg tatttatctt tctctctagg caaatattca tatataaatt 660
caatttgcca tgtgcatgat catctttgtt gggaattata aaacttttaa tttaatgagt 720
agatgtcagt atgtgcttca agtgatgaaa atttctttct caatattgcc aactttaatt 780
tctcgataat gcgatcctac aaaaaatctg ttttctatta gagtagccca agagatccaa 840
aagcacaaaa tcatagactt atctctctct tttttttttt ttttttgtta aaaggcttaa 900
gacatagagt cgtagattta tcttcattac aaaaataaac atgaacattc ttctaatatt 960

gatttactca gttttctgca acagtttcac tttaccatat ctgcataaat gacgacgttt 1020
taccatttgt agaaattttg cctttggatc attgactttt atatatgcag ttgttaaaat 1080
gtttctatct ataaagtaca aaagaaagag agaaagacaa atttctgatc atttctttcc 1140
aaacacctct cgataattgt tagtttgttt ttcataggtt ttttgctaaa tagcccttcg 1200
acttaaagaa aaaaaaatat cttctaacat aaaaacatac aaacaaataa tcttttcaaa 1260
ataaacccaa atatagccgt caaccatata gcaacgaccc aacaattgtc ctttgtacgt 1320
aaaacggtaa aagccacatt ttcttct 1347

<210> 22
<211> 1389
<212> DNA
<213> Arabidopsis thaliana

<400> 22
ggtggaagat tgttatcaca aagatcaaaa tacactgtgt tgtgaagctt gaggaaaaac 60
aagactttaa ataaaaccaa agtaaaataa ctcaacgtgg ctttttaatt taataataga 120
attcacgtgg cttttatttt tatatatatt ccacgttcct aattacaaag aattcaacgc 180
tatttttaat tttattcttg tcttcattag tttctgtgta tgtgaaacgc ctataaatta 240
tgattatccc actcttaatc ttgccaaaag aaagactaag aaaaatgata tttttaaaaa 300
agtgaaaaaa cgaaaagttt attattgaaa tatatctata atggaatcca ctaatgctgg 360
taaatccaac tattaatcag tttaacttac tacaatatta tgaaatagaa gtataatcat 420
tagagtataa ttaattatcc tcataggcca tagtccacgt attgtgaact actttgctga 480
tataaataaa taatgcgatg ttcactacac tactaccaaa tcgtatctgt tagacttggt 540
atttatttat actataagat aatattagcg tgctacatga gaaaattaaa tacatatata 600
tgattgtact tgtacagaac tgacaaccac atgtgtcacg accctaaaac tcaatgatcg 660
gctataatta aattttcgtt cggcagtaaa ttcaatatca aacctttag cttgacttaa 720
ttttctagtt tactttttaa ccaatccact ttttcctaat actaaaaata tgaaaaaatc 780
ttgtaaaata ataaacctga agtctctctc taaatattga tattttaacg tctctataga 840
taattagtag acgacagctt gacgatagac gagtcagatt cgattcgatt cgatccgatc 900
cgattagaga atataatatt gagaggggac acattgacat tgtttatccg gaagtaacgc 960
gacctagctg cccatgtatc atccatttcg ttgctcttcg atctattcct aaaacgtgct 1020
acatagaggg atactattaa taaaacaaaa ctgatttgaa gtaagaaaaa taactacaca 1080
aaaaaactta tagttctgtt ataaatagaa ataatgtcta gtgtcatatt tcattactgc 1140
tttgggccac ataatgaaag ctgagtcatg tctttcgacc ccaaataatt tttttttaat 1200
ggtaattggt aaattaatta ttattcttta cgaagtattt atataatctg ctgatatcat 1260
taaagtaaag agcaatttga caaggcgctc acataaaagc tttatattag aaagggaaat 1320
gacaaggaat aagacataaa caagagagaa agcatgtaat taaaagagaa gaaaacaaaa 1380

gacttgtct 1389

<210> 23
<211> 2472
<212> DNA
<213> Arabidopsis thaliana

<400> 23
caccttagta tagatttaga atatatttgg atctaaacct cacaatatat atattattca 60

tattggatat ggctatcctt tcatagttgc aattggccgc atagtattca taatttgtct 120

tttctttagg atgtaaaatt tgtattcatc cacaaatcat tttcagaaca aatgcattct 180

tgttttgaag tttaaaaaga gaaacagagc ttaatagtaa tacagaaagt tttttaagtt 240

agacaaattc actctatcaa tttgactttt gatctcaaat ttgtgttaaa ttttttttggg 300

acaactctaa cgttgaaatc atacggctga aatatcactc ccatttaaaa ataatattcc 360

aaactagtct atctgtccat cttgtaaccc ctgatatacg gaattatttg ataagtccaa 420

ccatttgaga ttacaaaaac aattatcaca tgaaaatgat atcataactt tgtcttcgtg 480

tgagactgtg tagattaact tatgttttag aaataattgt tatataagtc tgccccgtga 540

caaacggatt aatgaaggat aataagatat gttcacatgg ccaacgtgaa agcataaaaa 600

acaattattt atttttagat atattttaat tttaatttga tttaaatatt gtacctggtt 660

ttcaaataac cacatctaag gcgttagcta gtaatttatg tcattgaaaa aagccagaaa 720

gaaacaaaag aacattcttc atgtaattgg cttctgtgaa aaatgaattt aaaaaaatac 780

ttctaacaag ttgataaata ggtctggaca taaaaatcgg aacccgagta ctctaatcgg 840

cgtcaaccta acattttgaa ctgaatccca acccaaaaaa ttagatttat cctactaaat 900

cctatattcc ttatttcttt tacttataat ccctaacttg aactccatct aaatctgaat 960

ctgaacatac tctcaaaata agtacatact aataacttta gacttgaata tccaaatttt 1020

ttacttaaat acccgcaata taagtacata cacaaatttt tatccaaaac ctagatattt 1080

acctaaattg ttttgggtat ttgcataata ttttgagtat ttgtgtaccc aaaaagaatc 1140

gggtatttaa tagttctaaa aatttataca tgattttatc caaactcgaa aaaaccaaaa 1200

ccgaaatcga accattttta tatttaccct attagatttt attttatttt tcctatttcg 1260

aacacggatg gttcctacca gattctgcct taagtttctt aatgttcaga cctattgata 1320

agtatgagtt ttatcaatga gcatcaagta aaagtgagag tcttgattct gctttaaaac 1380

atgaaagttt ttattgtata atatggatat cgaagaggct aattcagata tataaaatta 1440

cataatacct gatttattag agtccatata atttacataa gactttccac gactaattta 1500

tatagttaaa atactatatt tgattatatt tttagtaatc gtgaagactc gttcatatat 1560

tgatatacag tataacctct ataaattaat actctataaa ttaataacct ctataaatta 1620

ataaattatt ccggtcccga gttaggacca gtgtaaaaaa tgacataaat cgataaatta 1680

ataagataat attttttttg aaaattctat gtaaatctat ggtcctatca atatcataaa 1740

ttaataattg tataaatgta tcaactatat atatatatat attatgtaaa aaaattcttt 1800

ataatatatt tattatactt ttttgcttaa attcatattt gttctttgtt tgattatagt 1860

tttcatattt tactgtatca aaacttttag tgttgttttc taaacattat tattatgttt 1920

tacttgtaac tggttctaga aatatatatt ctataatgaa ggaaatctta tagaaatttt 1980

taaaatgtta ccaaattagg aaaatctctc tataaattga taaatattaa tttatcgata 2040

aattaatacc tctctaaatt aataaaattt cgcagtccca acattactaa tttatagagg 2100

ttttactgta attcgtaaat tttgagagta gtttttaacc aacttacacc accaatttat 2160

tagagtacat attttgattt tcgatatgca atcttgttac caactaagtt gttaattagg 2220

accatatact cttagaaatt gccagctaat ttgaacaatg accatttaag tgttttcaaa 2280

taatcaaatc atatccatat aaaaataatt agtcaactaa tcccctatta agaaaaatca 2340

acaattaaaa cattctcatt tcctcctaat gaatacccta taaataccca ctaaaaactt 2400

acatttctcc caccaatctc aaagcaaatt aaatacacta ctacttcttg agcttttaaa 2460

ctacacaaac aa 2472

<210> 24
<211> 1749
<212> DNA
<213> Arabidopsis thaliana

<400> 24
tctctattat tgattttttt aggcagaaaa attgtctaga ttttatttat atgcgagttc 60

aaaatggggt tttctaaata aaatttttaa aaaaaaaaac ttctcaatgt ttaagataaa 120

ataaaaataa aacaaattat cggctacaat atctaaagtc tatgaaactt tatttatatg 180

attaagtatg acatgtcgta tttctctcat ttagctttgg gattaagata cagctgcaca 240

taataaacat cctacaagtt ttaaaaccgt ctaatcagag atatttctga tgcatataat 300

attgtctatt gtaaaatcat ctttcaaact ataaaataag agtattccac tagttatgtg 360

tcatcaaaga ttaggtcaga cctatgataa ttatcaatta tttaattcag attgttataa 420

tcgactacaa ctcatatcaa cttataaatt gcctaactta tatgattcaa tatattattc 480

attaaattaa tgttatgtga ctgtataaca tcagccaagt tgtgaacaca aaaagcttga 540

taattgatat aatcctcaac aattttataa ccgatacctt ttttattttc ttcacaccaa 600

acggtagcct tttttataat atctcttcgt cggagaataa taagacaaga tatttgatat 660

tctcatacag aaatatataa atctagttcc tatatatact ttaattaata acagctataa 720

aatctatatc ataaatcaca gtgcaatgat atgtcagatc atttgtgatt agagctccaa 780

tcatatacta gtaaattctt aaatccatct ccttgttatc gattttgtgt gtttcccaac 840

aaatatgtta agtgcaaata ttaaaacctc caaccaaaca aaatatatca caatctatga 900

ataatattga caactactaa atagatcgta agttttagtt tatgcaagac tatacataca 960

gtagttttgt aaagttactt atattggcaa acaaaatata attctggtat tcagtttgaa 1020

aacatacgtt aatttacaca atatagtttt gtaacaacag atttaaactc catattttgt 1080

aaacaattgt aggctataga ttaaaatata gaaaactcat acacgactac tcatactgga 1140

cccagtcata ttgtaaggta aactgcgtat taaacgtcgt gtaatcagtc aattatgatt 1200

caccatgaac agcaatacag cataatatta tagtagtaaa atactttaaa atcctttttt 1260

ttcgtttgac aaagcgctat actaaaaaat atagcaaact caagtataca ctaaaataaa 1320

attagtacat ttgctagtct tcacctctag aagacaagag tatgtcgttt tcttgacaaa 1380

gacgatttgg ttttggtgag ttattatagt aaataaatat aatactaata aaaatgtatt 1440

tattataatc tatagattag agagacagct aaacgctaat gtgtagttta tgcgcgtacg 1500

acgcggttaa ttttgttttc tctttttttt ttaaagtaaa caaagatatt aaaataaaat 1560

aaaacacgag accgtgtcac taactaacag agacgtagct acacatatat aaagagatcg 1620

agaggtcttc atttctcaca agccacaaca acaccaatca cacttctcaa aaaagatcag 1680

agatttctag atccttcttc ttctcctgca aattcatcta cctgaatctt ccaactttat 1740

aaatcgatc 1749

<210> 25
<211> 39
<212> DNA
<213> artificial

<220>
<223> linker

<400> 25
cggaattcct aggcttctgc ccgggcttct gggtacccc 39

<210> 26
<211> 39
<212> DNA
<213> artificial

<220>
<223> linker

<400> 26
ggggtaccca gaagcccggg cagaagccta ggaattccg 39

<210> 27
<211> 24
<212> DNA
<213> artificial

<220>
<223> primer

<400> 27
gacccgggac tgcaacgaag tgta 24

<210> 28
<211> 26
<212> DNA
<213> artificial

<220>
<223> primer

<400> 28
caggtacctt tttgtttgta attctg 26

<210> 29
<211> 3211
<212> DNA
<213> Arabidopsis thaliana

<400> 29
atgatagctt acatagaacc atacttcttg gaaaacgacg ccgaggctgc ctctgccgcc 60
accgccgccg gaaaatctac ggatggtgtt tctgagtcac ttaacatcca aggagaaatc 120
ttatgtggtg gagctgcggc ggatatcgcc gggagagatt ttggcggcat gaactgtgtg 180
aagcctcttg ctgtggtgag accagtggga ccggaagata tcgccggagc ggtgaaagcg 240
gctctgaggt cagataaact aacggtggcg gcgcgtggaa acggccattc tatcaacggt 300
caagccatgg cggaaggagg actcgttgtc gatatgagta ccacggcgga gaatcatttc 360
gaggttggtt atttatccgg cggtgatgcc acggcgtttg ttgatgtctc cggaggggca 420
ttatgggaag atgtattgaa acggtgcgtt tcggagtacg gtttggctcc gaggtcttgg 480
actgattatc ttgggttaac ggtgggaggt acgttgtcaa atgccggcgt tagtggtcaa 540
gcgttccgtt acggaccaca gacgtcaaat gtaacggagt tggacgtcgt tacgggaaat 600
ggtgacgtcg ttacttgctc ggagattgag aattcagagc tattcttctc tgttttaggt 660
ggtcttggtc agtttggtat catcaccaga gctagggttt tgctacagcc agctcctgat 720
atggtgaata cttaaaacca acaatataaa taacaatctc agttatatat atatatattt 780
tctctaatcc aatcaaaaat aagatatttg gtccataata taaatgattg ttgtgttagg 840
tgagatggat aagagtagta tacaccgagt tcgatgagtt cactcaagac gccgagtggc 900
tagtaagtca gaagaacgag tcatcgttcg attacgtgga aggattcgtg tttgtcaacg 960
gtgctgaccc ggttaacgga tggccaacag ttcccctcca cccggaccac gagtttgacc 1020
cgacccgact accacaatct tgcgggtcgg ttctttattg cctcgaactc ggtcttcact 1080
acagagactc cgattccaac tcaaccattg acaaggtaat aataactttg agaaacttta 1140
taacattttt cagaaattca agaaccgttc atcttttatg atctaacggc ggtggaagat 1200
tctgatgttc tagaaacttt gtttgaccga aattgacctt agattgaagt gtgaagttga 1260
cccgttttat ttcactaact gttatacgac acgtagttca tataggaccg ttttcagatt 1320
tctcgacctc catgattaca caaacataca attcaaaaaa cagtaaaaag atgataataa 1380
taataatata tggtttagtt aaggaaatat aatagggtgg gaaagggaat tatacagtct 1440
cttgtctgac tgcataatat gaaactgacg agacattgtg taatgtatct tcgattttgg 1500
attgtctgac atgaaaaaaa tatttatttg cttctctcta atgcccttgt cgtaaccacg 1560
ttattacgaa aaggacattt gtcttcgttt tctttttctt ttcttttttt ttgttgcttt 1620
tgttgtcttt ctcatgaaac acatatttta agatcacttt gccttttttct actcaattat 1680
ttagattaaa ccaacacgtg tgacgtgtcc attggtcgtg cggtatggga cgtaaggttg 1740

```
aaatcgtaat tgtagcatgt aaacgtttct gtagtaaaac attgatgata tgatttcaaa   1800
cggtcccggc taaaatctgg ccatcgtttt atatggaatc atctatgtat gtaccgaaat   1860
acccccctgac tgattttttt ccattttttt gtgtagaggg tggagagatt gatcggacgg   1920
ctaagattta atgaaggatt aagattcgag gtagatctgc cgtacgttga ctttttacta   1980
cgagtcaaac ggtcagaaga aatcgcgaag gagaacggta cgtgggaaac gcctcacccct   2040
tggctcaacc tcttcgtgtc gaagcgagac atcggagatt tcaatcggac ggtgttcaaa   2100
gaacttgtca agaacggagt caatggtcca atgcttgtgt acccactctt gcgaagcagg   2160
tgaatattgc tctctcttcc tctcttttaa ctaggaccca tctttttatt ttgggttaga   2220
caaggcacct atctaaaaga ctaaaagaag atcggtctag tttagtttta tggcatgtgt   2280
ttatcacgtg tgatgattag tcgtgcatgc ttaaactaaa aaaaggctcc acaagtcgca   2340
agtcgtgtga tcaacaaatt gtcgccaatg tggcacacgt gtctttcttc agtcccctcg   2400
tcattttttt tacccgtacg gggttttaag tacaataaaa gttggaattt agtgtggtcg   2460
tttagatttt gtaggcgaga taaaaaaaag aatactaaac taatacgatg ccgtattagg   2520
tattacggtt gggtgggtga cggatatgta ttgtaaccgt cgttaaggtt agcgtcatat   2580
agggaaagag atgaaatttg tagggaccca atatgaacta acgttaaatt ttttatttta   2640
catttctaaa atagcctttt gtaggttact gaagggtagt ttcgtcttta tatgttttac   2700
tttatgtgga aaatgagatt tgctggtaca aatagtagac gtaagaaatg aaaccaatcg   2760
tgagcaaagg gccaccaaaa tgtttatttt ttattctccg attttttat ggaaaatgtc   2820
ttttgttcca tttagattta gtgggtattt gttttataat atgaatgatt aaataataat   2880
ttggttggtt tttatcaggt gggatgatcg gacgtccgtg gttataccgg aagaaggaga   2940
gatattctac attgtggcat tgcttcggtt cgtgccgccg tgtgcgaaag tctcttcggt   3000
agagaaaatg gtagctcaaa accaagagat cgttcattgg tgtgtcaaaa acggaattga   3060
ttacaaattg tatcttcctc attacaagtc tcaagaggaa tggattcgcc attttggaaa   3120
ccgatggtcg agatttgttg ataggaaagc tatgtttgat cccatggcta tactttcacc   3180
gggtcaaaag attttcaata ggtctctttg a                                  3211
```